# EUROPÄISCHE PATENTSCHRIFT

(11) **EP 2 512 349 B1**
(45) Veröffentlichungstag und Bekanntmachung des Hinweises auf die Patenterteilung: **25.09.2019**
(21) Anmeldenummer: 10795344.0
(22) Anmeldetag: 17.12.2010
(51) Int. Cl.: A61B 17/11, A61B 18/14, A61B 17/064, A61B 17/115

(54) **CHIRURGISCHES SYSTEM ZUM VERBINDEN VON KÖRPERGEWEBE UND ZUM ABTRENNEN ÜBERSTEHENDEN GEWEBES**
SURGICAL SYSTEM FOR BONDING BODILY TISSUE AND FOR CUTTING OFF PROTRUDING TISSUE
SYSTÈME CHIRURGICAL POUR RELIER DES TISSUS CORPORELS ET POUR SECTIONNER UN TISSU EN SAILLIE

(30) Priorität: 17.12.2009 DE 102009059195
(43) Veröffentlichungstag der Anmeldung: 24.10.2012
(73) Patentinhaber: Aesculap AG, 78532 Tuttlingen (DE)
(72) Erfinder: WEIßHAUPT, Dieter, 78194 Immendingen (DE); KELLER, Anton, 78589 Dürbheim (DE); ROTHWEILER, Christoph, 78166 Donaueschingen (DE)
(74) Vertreter: Winter, Brandl, Fürniss, Hübner, Röss, Kaiser, Polte - Partnerschaft mbB
(86) Internationale Anmeldenummer: PCT/EP2010/070017
(87) Internationale Veröffentlichungsnummer: WO 2011/083026

(56) Entgegenhaltungen:
- EP-A1- 1 815 805
- EP-A1- 2 030 578
- EP-A2- 1 935 348
- EP-A2- 2 111 812
- WO-A1-2005/004734
- WO-A1-2006/021269
- WO-A1-2009/022614
- US-A1- 2003 045 811
- US-A1- 2003 069 571
- US-A1- 2006 064 086
- US-A1- 2008 243 121
- US-B1- 6 395 002

## Beschreibung

Die vorliegende Erfindung betrifft ein chirurgisches System zum Verbinden von Körpergewebe, umfassend ein chirurgisches Instrument mit einer Verbindungseinrichtung zum Verbinden von Körpergewebe, welche Verbindungseinrichtung zwei relativ zueinander bewegbare Werkzeugelemente umfasst, wobei das Instrument eine Schneideinrichtung mit einem Schneidenelement zum Durchtrennen von Gewebe umfasst und das Schneidelement relativ zu mindestens einem der Werkzeugelemente bewegbar angeordnet ist.

Des Weiteren wird ein beispielhaftes Verfahren zum Abtrennen überstehenden Gewebes von zwei zuvor miteinander zu einem einzigen schlauchförmigen Gewebeteil verbundenen schlauchförmigen Körpergewebeteilen beschrieben.

Chirurgische Instrumente chirurgischer Systeme der eingangs beschriebenen Art sind beispielsweise bekannt in Form von Koagulationsinstrumenten, bei denen mit einer entsprechend vorgesehenen Schneideinrichtung überstehendes, koaguliertes Gewebe abgetrennt werden kann. Des Weiteren sind Klammernahtgeräte bekannt, mit denen schlauchförmige Gewebeteile, beispielsweise zur Herstellung von End-zu-End-Anastomosen, ringförmig miteinander verbunden werden können, und zwar durch Anbringen von Klammern. Dabei ist es üblich, die Klammernahtgeräte mit Ringmessern auszustatten, also mit Schneiden, die in sich ringförmig geschlossene Schneidkanten aufweisen. Nachteilig bei allen bekannten chirurgischen Systemen ist, dass teilweise sehr hohe Kräfte zum Durchtrennen des Körpergewebes benötigt werden.

Es ist daher Aufgabe der vorliegenden Erfindung, ein chirurgisches System sowie ein Verfahren der eingangs beschriebenen Art so zu verbessern, das Körpergewebe mit geringeren Schnittkräften durchtrennbar ist.

Die dem Anmeldungsgegenstand zugrundeliegende Aufgabe wird durch die im Anspruch 1 angegebenen Merkmale gelöst, besondere Ausführungsformen der Erfindung sind in den Unteransprüchen angegeben.

Diese Aufgabe wird bei einem chirurgischen System der eingangs beschriebenen Art erfindungsgemäß dadurch gelöst, dass das Schneidelement eine Schneidkante aufweist, welche eine relativ zu einer vom Instrument im Bereich der Verbindungseinrichtung definierten Längsachse geneigte Schneidebene definiert.

Durch ein Schneidelement mit geneigter Schneidkante bezogen auf die Längsachse der Verbindungseinrichtung kann insbesondere eine Flächenpressung zwischen dem Schneidelement und dem Gewebe vermieden werden. Vielmehr ist aufgrund der Neigung eine punktuelle Krafteinleitung im Moment oder Zeitpunkt des Anschnitts mit einem solchen Schneidelement möglich. Damit sind die Kräfte, die zur Durchtrennung des Gewebes, insbesondere im Anschnittmoment, benötigt werden, deutlich niedriger als bei einer flächigen Durchtrennung. Eine flächige Durchtrennung liegt insbesondere vor, wenn die Schneidkante eine Schneidebene definiert, die senkrecht zur Instrumentenlängsachse im Bereich der Verbindungseinrichtung verläuft. Durch die geneigte Schneidkante kann insbesondere auch bei ringförmigen Schneiden der Schnittpunkt an der Schneidkante entlangwandern, und zwar ausgehend von einem Anschnittbereich der Schneidkante, welcher insbesondere vor dem in Kontakt Treten mit dem zu beschneidenden Gewebe von diesem den geringsten Abstand der gesamten Schneidkante aufweist. Das Schneidelement in der beschriebenen Weise auszubilden hat sowohl bei einem rein mechanischen Einsatz als aber auch bei einer monopolaren oder bipolaren Ausgestaltung Vorteile. Insbesondere im Falle einer monopolaren oder bipolaren Schneideinrichtung ermöglicht die lediglich punktuelle Anlage der Schneidkante am zu durchtrennenden Gewebe und die damit verbundene punktuelle Stromkonzentration einen problemlosen Anschnitt und insgesamt einen homogenen Schnittverlauf. Dadurch wird die erforderliche Energie zum Durchführen eines Schnitts deutlich herabgesetzt, und so zwar sowohl beim mechanischen als auch beim elektrischen Durchtrennen von Körpergewebe.

EP 2 030 578 A1 offenbart ein mechanisches Rotationsschneidsystem zur Verwendung mit einer chirurgischen Klammervorrichtung. Das Rotationsschneidsystem umfasst eine Messerklinge mit einer distalen Schneidkante und einer proximalen Basis.

EP 1 815 805 A1 offenbart ein mechanisches Klammerinstrument zur Anastomose.

WO 2006/021269 A1 offenbart ein chirurgisches Instrument, insbesondere für laparoskopische oder dergleichen minimalinvasive Eingriffe, umfassend - mindestens zwei gegeneinander bewegbare Klemmteile mit Klemmflächen zum Fassen von Gewebe in einem Schließzustand der Klemmflächen und - eine Schneideinrichtung mit einer Schneidkante, die in einer Schneidrichtung relativ zu den Klemmteilen zum Schneiden des gefassten Gewebes bewegbar ist.

WO 2005/004734 A1 offenbart eine endoskopische bipolare elektrochirurgische Zange zum Abdichten und / oder Schneiden von Gewebe. Die Zange weist eine Antriebsanordnung für eines der Backenelemente relativ zu dem anderen Klemmbackenelement auf um es von einer ersten Position zu bewegen, wobei die bewegliche Klemmbackenelemente in einer beabstandeten Beziehung relativ zueinander zu einer zweiten Position angeordnet sind, wobei die Backenelemente zur Manipulation von Gewebe näher zusammengebracht werden.

US 6 395 002 B1 offenbart ein elektrochirurgisches Instrument für die Ohrchirurgie, insbesondere zur Verwendung bei der Myringotomy (Loch/Einschnitt in das Trommelfell) und umfasst unter anderem ein elektrisch leitendes Element an einem ersten und einem zweiten Ende.

US 2003/069571 A1 offenbart ein Instrument und ein Verfahren zum Abdichten und Verbinden oder dem hämostatischen Teilen von Gewebe und ist besonders für die laparoskopische und endoskopische Chirurgie geeignet. Das Gerät macht Gebrauch von der gesteuerten Anwendung einer Kombination aus Wärme und Druck um benachbartes Gewebe zu versiegeln, zu verbinden oder Gewebe zu anastomosieren, wobei Gewebe für eine bestimmte, optimale Zeit und bei einer optimalen Temperatur unter optimalen Druck erhitzt wird um Gewebeabdichtung zu maximieren, während Schäden minimiert werden.

Besonders einfach ausbilden lässt sich das chirurgische System, wenn die Schneideinrichtung in Form einer mechanischen Schneideinrichtung ausgebildet ist. Beispielsweise kann die Schneidkante in Form einer geschärften Schneidkante ausgebildet sein. Diese kann beispielsweise durch Anschleifen eines geeigneten, vorzugweise gehärteten Instrumentenstahls ausgebildet sein.

Günstig kann es ferner sein, wenn die Schneideinrichtung in Form einer HF-Schneideinrichtung ausgebildet ist. Eine HF-Schneideinrichtung ermöglicht das Durchtrennen von Gewebe mittels eines HF-Stroms. Mit diesem kann insbesondere beim Durchtrennen des Gewebes gleichzeitig eine Koagulation desselben erreicht werden, wodurch unerwünschte Blutungen vermieden werden können. Optional kann die HF-Schneideinrichtung auch in Kombination mit einer mechanischen Schneideinrichtung vorgesehen sein.

Eine besonders, nicht erfindungsgemäße, Ausgestaltung eines chirurgischen Systems kann, insbesondere beim Vorsehen einer HF-Schneideinrichtung, dadurch erreicht werden, dass die Schneideinrichtung in Form einer monopolaren Schneideinrichtung ausgebildet ist.

Auf einfache Weise kann ein besonders sauberer und definierter Schnitt insbesondere dann realisiert werden, wenn die Schneideinrichtung in Form einer bipolaren Schneideinrichtung ausgebildet ist. Dies bedeutet, dass erfindungsgemäß das Schneidelement eine Elektrode bildet und am Instrument eine entsprechende Gegenelektrode vorgesehen ist, wobei der HF-Strom zwischen der Elektrode und der Gegenelektrode durch die zu verbindenden Gewebeteile fließt.

Insbesondere zum Beschneiden von miteinander verbundenen schlauchförmigen Gewebeteilen, beispielsweise nach dem Herstellen einer End-zu-End-Anastomose, ist es vorteilhaft, wenn die Schneidkante ringförmig geschlossen ausgebildet ist. Dadurch kann ein beispielsweise kreisförmiger oder ovaler Schnitt je nach Bedarf auf einfache und sichere Weise von einem Operateur realisiert werden, und zwar sowohl bei einer mechanischen oder HF- oder einer kombinierten mechanischen/HF-Schneideinrichtung.

Um das Schneidelement, monopolar oder bipolar, in definierter Weise mit einem Schneidstrom, beispielsweise einem HF-Strom beaufschlagen zu können, ist es vorteilhaft, wenn das Instrument mindestens einen mit der Schneideinrichtung elektrisch leitenden verbundenen Schneidanschluss aufweist.

Vorzugsweise ist der mindestens eine Schneidanschluss elektrisch leitend mit dem Schneidelement verbunden. Er kann, insbesondere bei einer bipolaren Schneideinrichtung, auch mit einer entsprechenden, am Instrument vorgesehenen Gegenelektrode verbunden sein. Günstig ist es dabei insbesondere, wenn zwei entsprechende Schneidanschlüsse vorgesehen sind.

Um Gewebe mit dem chirurgischen System auf einfache Weise miteinander verbinden zu können, ist es vorteilhaft, wenn die Werkzeugelemente jeweils eine Elektrode umfassen, die in einer Annäherungsstellung der Werkzeugelemente einen minimalen Abstand voneinander definieren, einander gegenüberliegen und aufeinander zuweisen. Durch entsprechende Bestromung der Elektroden können beispielsweise zwei miteinander zu verbindende Gewebeteile einfach verbunden werden, was auch als Verschweißen oder Versiegeln bezeichnet werden kann. Dabei ist es insbesondere wünschenswert, wenn es nicht zu einer Zerstörung der beteiligten Zellen kommt.

Günstigerweise ist mindestens eine der Elektroden als HF-Elektrode ausgebildet. Dies ermöglicht es, eine oder beide Elektroden mit einem HF-Strom zu beaufschlagen, der insbesondere zum Verbinden von Gewebeteilen, insbesondere Körpergewebeteilen eines Patienten, geeignet ist.

Gemäß einer weiteren bevorzugten Ausführungsform kann vorgesehen sein, dass mindestens eine der Elektroden in mindestens zwei Elektrodensegmente unterteilt ist und das die mindestens zwei Elektrodensegmente gegeneinander elektrisch isoliert sind. Die Unterteilung mindestens einer der HF-Elektroden in zwei oder mehr Elektrodensegmente hat insbesondere den Vorteil, dass sich die Verfahrensparameter zum Verbinden der miteinander zu verbindenden Gewebeteile signifikant leichter kontrollieren lassen als bei nicht unterteilten Elektroden. Je kleiner die Flächen, zwischen denen der HF-Strom zur Anwendung kommt, umso leichter lassen sich die Verfahrensparameter kontrollieren. Insbesondere haben die Temperatur, der Druck sowie die Gewebeimpedanz einen wesentlichen Einfluss auf das Verbindungsergebnis. Beispielsweise ist es so auch möglich, die Verfahrensparameter optimal auf die Gewebebeschaffenheit und insbesondere auch automatisch einzustellen. Darüber hinaus werden, anders als bei einem Klammernahtgerät, keine Klammern benötigt, die als Fremdkörper im Körper zurückbleiben. Insbesondere ermöglichen die die HF-Elektrode beziehungsweise die HF-Elektroden unterteilenden Elektrodensegmente eine segmentweise Bestromung der HF-Elektrode, so dass die miteinander zu verbindenden Gewebeteile segmentweise miteinander verschweißt oder versiegelt werden können. Eine durch die Segmentierung der HF-Elektroden möglich sequentielle Bestromung gestattet es, während des Verbindungs- oder Versiegelungsprozesses weniger Energie in die Gewebeteile einzubringen als bei vergleichbaren, unsegmentierten HF-Elektroden. Ferner hat die Segmentierung den Vorteil, dass zwischen durch HF-Bestromung verbundenen Bereichen der miteinander zu verbindenden Gewebeteile Gewebebereiche unverändert und im Wesentlichen ungeschädigt bleiben, so dass ausgehend von diesen neues Zellwachstum ermöglicht wird, welches zusätzlich zur durch den HF-Strom bewirkten Verbindung eine dauerhafte Verbindung der Gewebeteile durch ein Zusammenwachsen derselben ermöglicht.

Günstig kann es ferner sein, wenn das Schneidelement um die Längsachse verdrehbar ausgebildet ist. So kann bei Vorgabe einer Position des Instruments eine Position des Anschnitts mit dem Schneidelement in beliebiger und gewünschter Weise gewählt werden.

Um die Kontrollierbarkeit der Verfahrensparameter noch weiter verbessern zu können, ist es vorteilhaft, wenn jede der HF-Elektroden in mindestens zwei Elektrodensegmente unterteilt ist, die gegeneinander elektrisch isoliert sind. Mindestens zwei Elektrodensegmente im Sinne dieser Anmeldung bedeutet zwei oder mehr Elektrodensegmente, also insbesondere drei, vier, fünf, sechs, sieben, acht, neun, zehn, elf oder zwölf. Denkbar sind jedoch auch mehr, und zwar je nach Größe der Werkzeugelemente auch 20, 25, 30 oder 40 Elektrodensegmente.

Günstigerweise ist mindestens eine der HF-Elektroden in eine Mehrzahl von Elektrodensegmenten unterteilt. Unter einer Mehrzahl von Elektrodensegmenten sind im Sinne dieser Anmeldung mehr als zwei Elektrodensegmente zu verstehen, die eine noch weiter verbesserte Kontrollierbarkeit der Verfahrensparameter ermöglichen.

Um eine Bestromung der Elektrodensegemente auf einfach und sichere Weise gezielt vornehmen zu können, ist es günstig, wenn jedes Elektrodensegment mit einem Anschlusskontakt elektrisch leitend verbunden ist.

Vorteilhaft ist es, wenn die Werkzeugelemente jeweils eine Werkzeugelementfläche aufweisen und wenn mindestens eine Werkzeugelementfläche eben ist. Diese Ausgestaltung erlaubt es, die Werkzeugelemente praktisch vorsprungsfrei auszubilden.

Vorzugsweise ist die Werkzeugelementfläche ringförmig ausgebildet. Damit können ringförmige Verbindungen, beispielsweise bei End-zu-End-Anastomosen von schlauchförmigen Gewebeteilen, auf einfach Weise hergestellt werden. Insbesondere auch das Zusammenwirken mit einem eine ringförmig in sich geschlossene Schneidkante aufweisenden Schneidelement ist so einfach zu realisieren.

Vorteilhaft ist es, wenn mindestens eine der Elektroden in sich geschlossen ringförmig ausgebildet ist. Selbstverständlich können auch alle Elektroden des Instruments in sich geschlossen ringförmig ausgebildet sein. Gewebe kann so einfach und sicher ringförmig miteinander verbunden werden, was insbesondere für End-zu-End-Anastomosen vorteilhaft ist.

Um Gewebe zwischen den beiden Werkzeugelementen fassen und gegebenenfalls während des Verbindungsprozesses halten zu können, ist es vorteilhaft, wenn die Werkzeugelemente relativ zueinander verschwenkbar und/oder verschiebbar ausgebildet sind. Insgesamt ist also eine bewegbare Anordnung der Werkzeugelemente relativ zueinander wünschenswert. Eine Verschwenkbeziehungsweise Verschiebbarkeit der Werkzeugelemente relativ zueinander kann insbesondere auch beim Entfernen des Instruments Vorteile haben. Zum Beispiel kann so ein Querschnitt des Instruments im Bereich eines oder beider Werkzeugelemente zum Entfernen des Instruments verringert werden.

Gemäß einer weiteren bevorzugten Ausführungsform kann vorgesehen sein, dass das Instrument einen Schaft aufweist, an dessen distalem Ende mindestens eines der Werkzeugelemente angeordnet oder ausbildet ist. Auf diese Weise kann das Instrument besonders kompakt ausgebildet werden. Ferner kann durch die Anordnung oder Ausbildung mindestens eines der Werkzeugelemente am distalen Ende des Schafts die Stabilität des Instruments insgesamt erhöht werden. Insbesondere ist es so auch möglich, eines der Werkzeugelemente auf einfache Weise relativ zum Schaft unbeweglich auszubilden.

Vorteilhaft ist es, wenn ein erstes Werkzeugelement eine in distaler oder im Wesentlichen in distaler Richtung weisende Randfläche des Schafts umfasst. Beispielsweise kann so auf einfache Weise ein distales Ende des Schafts gegen einen Gewebeteil gedrückt oder gehalten werden, der mit einem anderen Gewebeteil verbunden werden soll. Außerdem kann so auch einfach und sicher eine definierte Werkzeugelementfläche vorgegeben werden.

Bei einer weiteren bevorzugten Ausführungsform kann ferner vorgesehen sein, dass ein zweites Werkzeugelement ein in Schaftrichtung bewegbares und in Richtung auf das erste Werkzeugelement hin und von diesem weg bewegbares Elektrodenelement umfasst. Diese Ausgestaltung gestattet es beispielsweise, die beiden Werkzeugelemente relativ zueinander derart zu bewegen, dass miteinander zu verbindende Gewebeteile in definierter Weise zwischen ihnen gehalten und durch entsprechende HF-Strom-Beaufschlagung miteinander verbunden werden können.

Damit die Werkzeugelemente auf einfache Weise relativ zueinander bewegt werden können, ist es günstig, wenn das Instrument eine Betätigungseinrichtung umfasst, zum Bewegen der Werkzeugelemente relativ zueinander.

Vorteilhaft ist es, wenn das Instrument eine Schneidbetätigungseinrichtung umfasst zum Bewegen des Schneidelements und mindestens eines der Werkzeugelemente relativ zueinander. Dies gestattet es einem Operateur mit dem Instrument zunächst Gewebeteile miteinander zu verbinden und dann die Gewebeteile, wahlweise direkt anschließend oder auch zu einem späteren Zeitpunkt, mit der Schneideinrichtung zu beschneiden. Insbesondere ist es auch denkbar, die Schneidbetätigungseinrichtung mit der Betätigungseinrichtung zu koppeln, beispielsweise derart, dass nach Abschluss des Verbindungsprozesses mit der Schneideinrichtung überstehende Gewebeteile automatisch abgetrennt werden.

Um die Handhabbarkeit des chirurgischen Instruments weiter zu verbessern, sind die Betätigungseinrichtung und/oder die Schneidbetätigungseinrichtung an einem proximalen Ende des Instruments angeordnet oder ausgebildet. Beispielsweise dann, wenn das Instrument einen Schaft aufweist, kann dieser durch eine Körperöffnung ins Innere des Patientenkörpers eingeführt werden, wobei dann die Werkzeugelemente relativ zueinander und/oder relativ zur Schneideinrichtung mittels der Betätigungseinrichtung beziehungsweise der Schneidbetätigungseinrichtung betätigbar sind, die dabei vorzugsweise noch aus dem Körper des Patienten herausragen. Insgesamt kann so auf einfache Weise ein endoskopisches oder minimalinvasives Instrument ausgebildet werden.

Die Handhabbarkeit des Instruments kann insbesondere dadurch für einen Operateur verbessert werden, dass die Betätigungseinrichtung und/oder die Schneidbetätigungseinrichtung zwei relativ zueinander verschwenkbare Betätigungsglieder umfasst, welche mit mindestens einem der Werkzeugelemente oder dem Schneidelement in Wirkverbindung stehen zum Übertragen einer Betätigungskraft zum Bewegen des mindestens einen Werkzeugelements relativ zum anderen Werkzeugelement oder des mindestens einen Werkzeugelements relativ zum Schneidelement. Die Betätigungsglieder können auch grundsätzlich nur relativ zueinander bewegbar ausgebildet sein, das heißt alternativ beispielsweise zu einer verschwenkbaren Anordnung können sie auch verschiebbar, oder verschwenk- und verschiebbar, zueinander angeordnet sein.

Um das HF-Instrument in gewünschter Weise mit einem HF-Strom beaufschlagen zu können, umfasst das chirurgische System vorzugsweise mindestens einen HF-Stormgenerator, welcher mit den HF-Elektroden und/oder dem Schneidelement wahlweise elektrisch leitend verbindbar ist. Insbesondere kann so der jeweils für das Verbinden beziehungsweise Durchtrennen von Gewebe optimale Strom eingestellt werden.

Die eingangs gestellte Aufgabe wird ferner bei einem Verfahren zum Abtrennen überstehenden Gewebes von zwei zuvor miteinander zu einem einzigen schlauchförmigen Gewebeteil verbundenen schlauchförmigen Körpergewebeteilen erfindungsgemäß dadurch gelöst, dass die zwei verbundenen Körpergewebeteile gehalten werden und dass die Körpergewebeteile ausgehend von einem Punkt umlaufend um eine vom einzigen schlauchförmigen Gewebeteil definierte Längsachse durchtrennt werden.

Durch das vorgeschlagene Verfahren werden deutlich geringere Schnittkräfte benötigt als dies bei Klingen der Fall ist, deren Schneidkante mit allen Punkten gleichzeitig auf dem zu durchtrennenden Gewebe auftreffen. Es kann so ein sauberer Anschnitt ausgehend vom genannten Punkt, der auch als Anschnittpunkt bezeichnet werden kann, ermöglicht werden, und zwar sowohl bei mechanischen als auch bei mono- oder bipolaren elektrischen Schneideinrichtung en.

Um überstehendes Gewebe bei End-zu-End-Anastomosen in definierter Weise entfernen zu können, ist es günstig, wenn ein ringförmiges Schneidelement verwendet wird.

Günstigerweise wird zum Abtrennen das Schneidelement mit einem Strom beaufschlagt. Insbesondere kann es sich dabei um einen HF-Strom handeln. Optional kann das Gewebe sowohl mechanisch als auch elektrochirurgisch durchtrennt werden, wobei die elektrochirurgische Vorgehensweise den Vorteil hat, dass mögliche, beim Durchtrennen von Gewebe auftretende Blutungen durch instantane Koagulation gestoppt werden können.

Die nachfolgende Beschreibung bevorzugter Ausführungsformen der Erfindung dient im Zusammenhang mit der Zeichnung der näheren Erläuterung. Es zeigen:
- Figur 1:: eine schematische Gesamtansicht eines chirurgischen Instruments zum Verbinden von Körpergewebeteilen;
- Figur 2:: eine vergrößerte, perspektivische, teilweise geschnittene und durchbrochene Ansicht des Bereichs A in Figur 1;
- Figur 3:: eine Längsschnittansicht des Instruments aus Figur 1 im Bereich A vor dem Verbinden zweier schlauchförmiger Gewebeteile;
- Figur 4:: eine Ansicht analog Figur 3 beim Verschweißen der Gewebeteile zur Herstellung einer End-zu-End-Anastomose;
- Figur 5:: eine Draufsicht auf eine Werkzeugelementfläche mit einer in vier Elektrodensegmente unterteilten HF-Elektrode;
- Figur 6:: (nicht beanspruchte Variante) eine perspektivische, schematische Ansicht eines zweiten Ausführungsbeispiels eines chirurgischen Instruments zum Verbinden von Körpergewebeteilen;
- Figur 7:: (nicht beanspruchte Variante) ist eine Draufsicht auf eine schematisch dargestellte Werkzeugelementfläche des Instruments aus Figur 6 in Richtung des Pfeils B;
- Figur 8:: eine schematische Ansicht ähnlich Figur 2 einer alternativen Ausgestaltung des Instruments in einer Gewebefassstellung;
- Figur 9:: eine Ansicht entsprechend Figur 8 des dort dargestellten Instruments mit teilweise abgeklapptem zweiten Werkzeugelement;
- Figur 10:: eine Schnittansicht längs Linie 10-10 in Figur 8;
- Figur 11:: eine schematische Schnittansicht ähnlich Figur 10 des in einer wie in Figur 9 dargestellten Stellung zusammengeklappten, zweiten Werkzeugelements;
- Figur 12:: eine alternative Ausführungsform eines zweiten Werkzeugelements in perspektivischer schematischer Darstellung;
- Figur 13:: eine Explosionsdarstellung eines Teils des in Figur 12 dargestellten zweiten Werkzeugelements;
- Figur 14:: eine Schnittansicht längs Linie 14-14 in Figur 12;
- Figur 15:: eine schematische Schnittansicht analog Figur 14 des dort dargestellten Ausführungsbeispiels mit teilweise abgeklapptem zweiten Werkzeugelement;
- Figur 16:: eine perspektivische schematische Darstellung ähnlich Figur 12 eines weiteren Ausführungsbeispiels eines zweiten Werkzeugelements;
- Figur 17:: eine vergrößerte Darstellung des zweiten Werkzeugelements aus Figur 16 in einer teilweise geneigten Stellung;
- Figur 18:: eine Schnittansicht längs Linie 18-18 in Figur 16; und
- Figur 19:: eine Ansicht analog Figur 18 bei teilweise geneigtem zweiten Werkzeugelement in einer Stellung, wie sie in Figur 17 dargestellt ist.

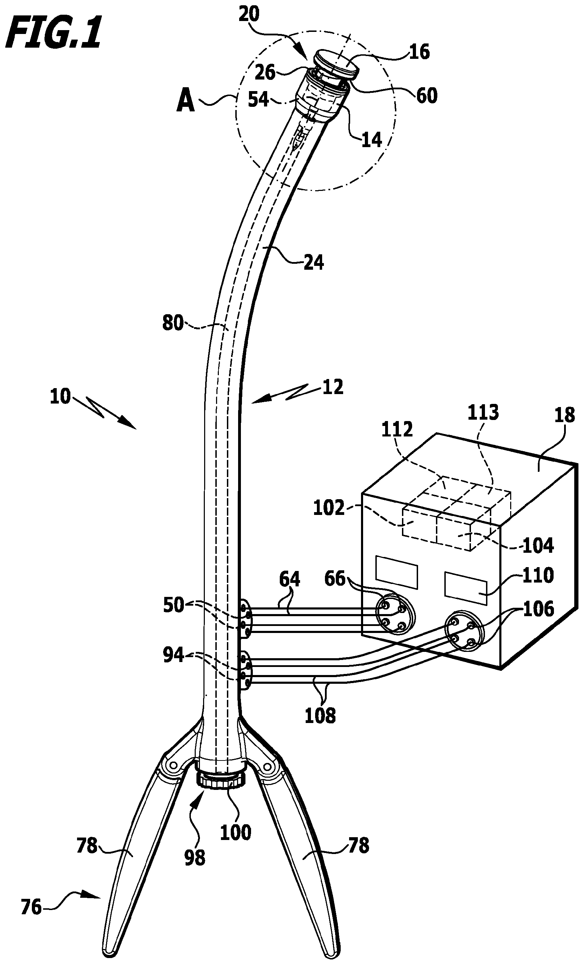

In Figur 1 ist ein chirurgisches System zum Verbinden von Körpergewebe schematisch dargestellt und insgesamt mit dem Bezugszeichen 10 bezeichnet. Es umfasst ein chirurgisches Instrument 12 mit zwei relativ zueinander bewegbaren Werkzeugelementen 14 und 16. Ferner umfasst das System 10 einen Stromgenerator in Form eines HF-Stromgenerators 18, welcher in einer weiter unten näher beschriebenen Weise mit dem Instrument 12 verbunden werden kann.

Die Werkzeugelemente 14 und 16 bilden einen Teil einer insgesamt mit dem Bezugszeichen 20 versehenen Verbindungseinrichtung zum Verbinden von Körpergewebe. Das erste Werkzeugelement 14 umfasst eine in distaler Richtung weisende Randfläche 22 eines langgestreckten, hülsenförmigen Schafts 24 des Instruments 12. Somit ist das erste Werkzeugelement an einem distalen Ende 26 des Instruments 12 angeordnet beziehungsweise ausgebildet.

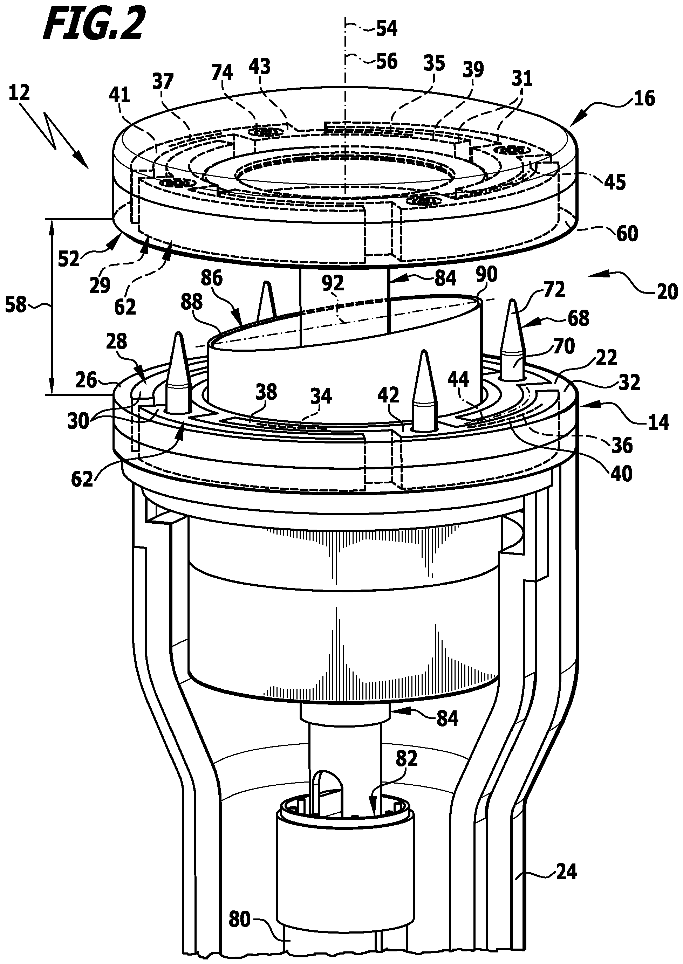

Das erste Werkzeugelement 14 umfasst eine HF-Elektrode 28. Sie ist in mindestens zwei, bei dem in den Figuren 2 bis 5 schematisch dargestellten Ausführungsbeispiel in vier Elektrodensegmente 30 unterteilt ist, die elektrisch gegeneinander isoliert sind. Die Elektrodensegmente 30 sind streifenförmig oder im Wesentlichen streifenförmig ausgebildet. Das erste Werkzeugelement 14 definiert eine Werkzeugelementfläche 32 derart, dass die HF-Elektrode 28 einen Teil derselben bildet. Insgesamt ist die Werkzeugelementfläche 32 eben und ringförmig ausgebildet.

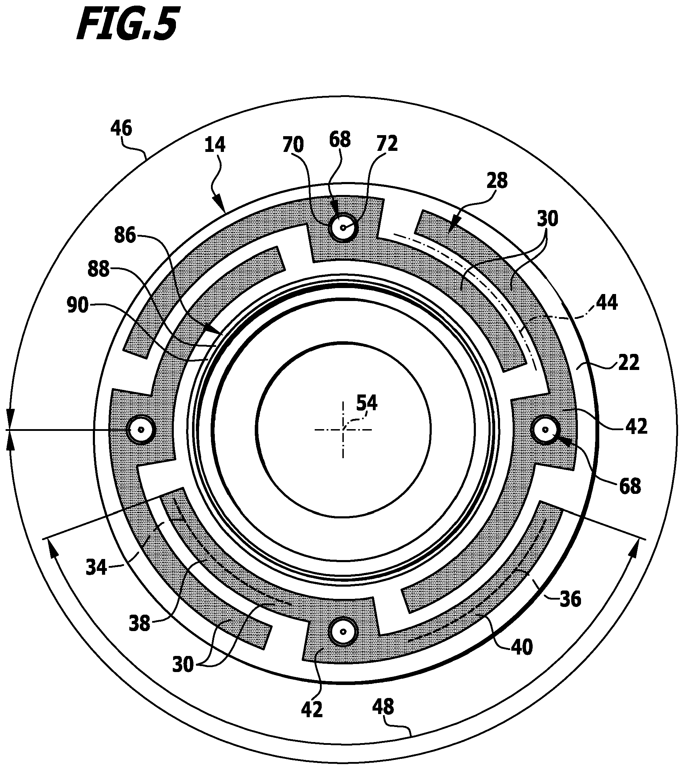

Die vier Elektrodensegmente 30 definieren zwei Elektrodenreihen 34 und 36. Jede Elektrodenreihe umfasst dabei jeweils einen Teil der vier Elektrodensegmente 30. Wie zum Beispiel in Figur 5 zu erkennen, weist jedes Elektrodensegment 30 einen ersten Elektrodensegmentabschnitt 38 auf, welcher einen Teil der ersten Elektrodenreihe 34 bildet, und einen zweiten Elektrodensegmentabschnitt 40, welcher einen Teil der zweiten Elektrodenreihe 36 bildet. Die beiden Elektrodenreihen 34 und 36 sind insgesamt gekrümmt ausgebildet, wobei die Elektrodensegmentabschnitte 38 und 40 jeweils elektrisch leitfähige Kreisringabschnitte definieren. Insgesamt sind die mindestens zwei Elektrodenreihen, die durch jeweils vier Elektrodensegmentabschnitte 38 beziehungsweise 40 definiert werden, in sich geschlossen ringförmig ausgebildet. Um die Elektrodensegmente 30 in gewünschter Weise kontaktieren zu können, ist jedes Elektrodensegment 30 mit einem Anschlusskontakt 42 elektrisch leitend verbunden, welcher in einem Verbindungsbereich zwischen den Elektrodensegmentabschnitten 38, 40 angeordnet ist. Zwischen den Elektrodenreihen bleiben auch noch nach dem Verbinden der Gewebeteile durch HF-Bestromung vollständig oder im Wesentlichen ungeschädigte Zellen erhalten, von denen neues Zellwachstum ausgehen kann. Dies ermöglicht zusätzlich zur Verbindung der Gewebeteile durch Verschweißen langfristig eine dauerhafte Verbindung der Gewebeteile durch Zusammenwachsen intakter Zellen.

Die HF-Elektrode 28 definiert eine zwischen den Elektrodensegmentabschnitten 38 und 40 verlaufende Elektrodenmittellinie 44. Aneinander angrenzende Elektrodensegmente 30 sind daher in einer von der Elektrodenmittellinie 44 definierten Richtung versetzt zueinander angeordnet. Insgesamt definiert die in vier Elektrodensegmente 30 unterteilte HF-Elektrode 28 eine Elektrodenlänge 46, wobei jede der vier Elektrodensegmente 30 eine Segmentlänge 48 definiert, welche kleiner ist als die Elektrodenlänge 46. Wie beispielhaft in Figur 5 dargestellt erstrecken sich die Elektrodensegmente 30 über einen Winkelbereich von etwa 140° und weisen damit eine Länge auf, die annähernd 40% der Elektrodenlänge 46 entspricht. Damit ist aber auch die Summe aller Segmentlängen 48 etwa um den Faktor 1,6 größer als die Elektrodenlänge 46.

Im Bereich eines proximalen Endes des Schafts 24 sind HF-Anschlusskontakte 50 angeordnet, die elektrisch leitend, zum Beispiel über im Schaft verlaufende Leitungen, mit den Elektrodensegmenten 30 verbunden sind. Vorzugsweise entspricht die Zahl der HF-Anschlusskontakte 50 der Zahl der Elektrodensegmente 30, also vier HF-Anschlusskontakte 50 für die vier Elektrodensegmente 30 des ersten Werkzeugelements 14.

Das zweite Werkzeugelement 16 ist im Wesentlichen scheibenförmig ausgebildet und umfasst ein Elektrodenelement 52, welches in Richtung auf das erste Werkzeugelement 14 hin und von diesem weg bewegbar ist, und zwar parallel zu einer Längsachse 54 des Schafts 24 im Bereich der Werkzeugelemente 14, 16, welche eine Schaftrichtung 56 definiert. Die Werkzeugelemente 14, 16 sind relativ zueinander verschiebbar angeordnet, das heißt ein Abstand 58 zwischen der Werkzeugelementfläche 32 des ersten Werkzeugelements 14 und einer Werkzeugelementfläche 60 des zweiten Werkzeugelements 16 ist veränderbar.

Das Elektrodenelement 52 umfasst eine HF-Elektrode 29, die in ihrem Aufbau der HF-Elektrode 28 entspricht. Dies bedeutet, dass sie ebenfalls vier Elektrodensegmente 31 umfasst, die nicht über die Werkzeugelementfläche 60 vorstehen. Es werden ebenfalls zwei Elektrodenreihen 35 und 37 definiert, wobei erste Elektrodensegmentabschnitte 39 die Elektrodenreihe 35 definieren und zweite Elektrodensegmentabschnitte 41 die Elektrodenreihe 37. Anschlusskontakte 43 sind ebenfalls vorgesehen, die jeweils einen Elektrodensegmentabschnitt 39 mit einem Elektrodensegmentabschnitt 41 leitend verbinden zur Ausbildung eines Elektrodensegments 31. Die HF-Elektroden 28 und 29 sind spiegelsymmetrisch zu einer senkrecht zur Längsachse 54 zwischen den Werkzeugelementflächen 32 und 60 verlaufenden Spiegelebene ausgebildet. Auf diese Weise werden Elektrodensegmentpaare 62 definiert von jeweils einem Elektrodensegment 30 und dem entsprechenden, gegenüberliegenden Elektrodensegment 31. Insgesamt umfasst das in den Figuren 1 bis 5 dargestellte Ausführungsbeispiel somit vier Elektrodensegmentpaare 62. Die Elektrodensegmente 30, 31 sind nicht nur geometrisch ähnlich, sondern gleich groß beziehungsweise im Wesentlichen gleich groß.

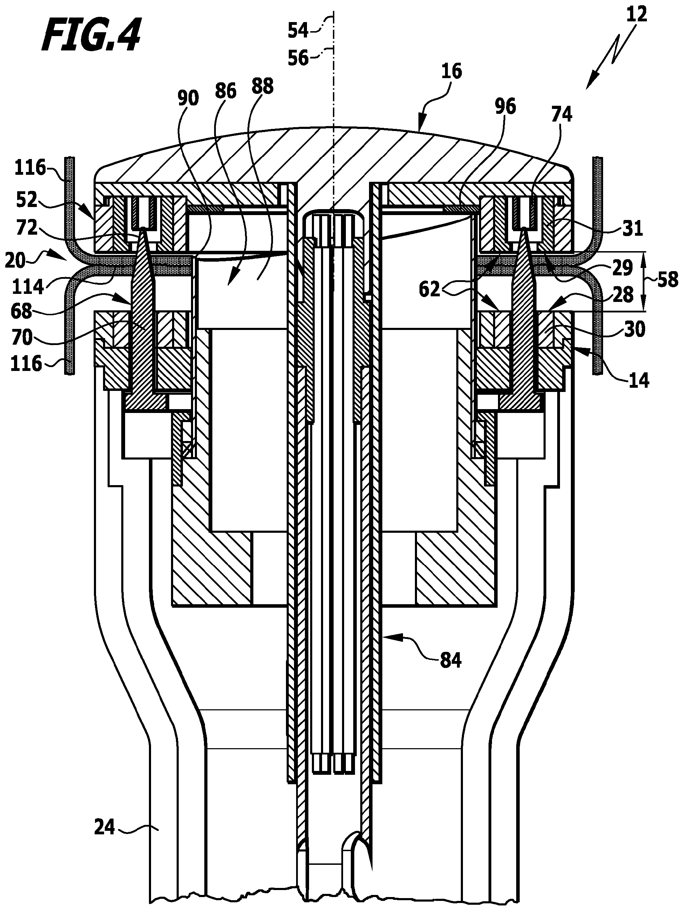

In einer Annäherungsstellung der Werkzeugelemente 14, 16 definieren die HF-Elektroden 28, 29 einen minimalen Abstand 58 voneinander. Die Annäherungsstellung ist schematisch in Figur 4 dargestellt. In der Annäherungsstellung liegen die HF-Elektroden 28 und 29 einander gegenüber und weisen aufeinander zu.

Die Elektrodensegmente 31 sind elektrisch leitend mit weiteren vier HF-Anschlusskontakten 50 verbindbar, von denen der Übersichtlichkeit wegen in Figur 1 lediglich zwei dargestellt sind. Mittels entsprechender Verbindungsleitungen 64 können die HF-Anschlusskontakte 50 mit entsprechenden Kontakten 66 des HF-Stromgenerators 18 verbunden werden. Wie bereits dargelegt, sind die HF-Anschlusskontakte 50 mit den Elektrodensegmenten 30 direkt elektrisch leitend verbunden. Um die HF-Anschlusskontakte 50 mit den Elektrodensegmenten 31 verbinden zu können, sind am Schaft 24 beziehungsweise am ersten Werkzeugelement 14 in Richtung auf das zweite Werkzeugelement 16 hin weisende Kontaktglieder 68 abstehend angeordnet, welche einen kurzen zylindrischen Abschnitt 70 und einen, ein freies Ende definierenden kegelförmigen Abschnitt 72 aufweisen. In einer Gewebeverbindungsstellung, wie sie beispielsweise schematisch in Figur 4 dargestellt ist, also in einer Stellung, in der sich die Werkzeugelemente 14 und 16 in der Annäherungsstellung befinden, ragen die freien Enden der Abschnitte 72 der Kontaktglieder 68 in entsprechende buchsenförmige Aufnahmen 74 des Elektrodenelements 52 hinein und stehen mit diesen elektrisch leitend in Kontakt. Die Kontaktglieder 68 wiederum sind längs des Schafts 24 über nicht dargestellte elektrische Leitungen mit den HF-Anschlusskontakten 50 verbunden. Die Aufnahmen 74 wiederum stehen mit den Anschlusskontakten 43 elektrisch leitend in Verbindung. Auf diese Weise kann in der Annäherungs- oder Gewebeverbindungsstellung auch ein elektrisch leitender Kontakt zwischen den HF-Anschlusskontakten 50 und den Elektrodensegmenten 31 hergestellt werden.

Selbstverständlich sind die Kontaktglieder 68, die die Elektrodensegmente 30 im Bereich von deren Anschlusskontakten 42 durchsetzen, gegenüber diesen isoliert, so dass keine Kurzschlüsse auftreten können. Zu diesem Zweck sind die Abschnitte 70 der Kontaktglieder 68 vorzugsweise mit einer elektrisch isolierenden Beschichtung oder Hülle versehen.

Um die Werkzeugelemente 14, 16 des Instruments 12 relativ zueinander bewegen zu können, ist eine Betätigungseinrichtung 76 an einem proximalen Ende oder Endbereich des Instruments 12 angeordnet. Die Betätigungseinrichtung 76 umfasst zwei relativ zueinander verschwenkbare Betätigungsglieder 78, welche mit einem im Inneren des Schafts bewegbar gelagerten Kraftübertragungsglied 80 beweglich gekoppelt sind, so dass dieses in Folge einer Verschwenkbewegung der Betätigungsglieder 78 in distaler beziehungsweise proximaler Richtung bewegbar ist.

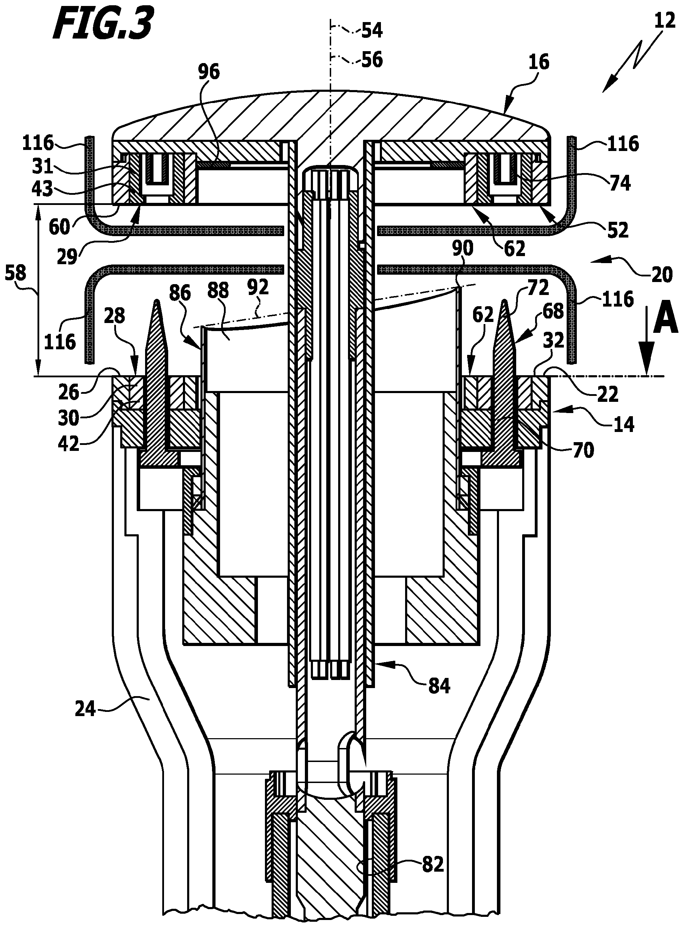

Das Kraftübertragungsglied 80 definiert an seinem distalen Ende eine sacklochförmige Aufnahme 82, in welche ein Halteglied 84 mit einem ersten freien Ende einführbar und in der Aufnahme 82 festlegbar ist. Das zweite freie Ende des im Wesentlichen stabförmigen Halteglieds 84 ist unbeweglich mit dem zweiten Werkzeugelement 16 verbunden. Auf diese Weise kann in Folge einer Verschiebung das Kraftübertragungsglied 80 in distaler Richtung das zweite Werkzeugelement 16 vom ersten Werkzeugelement 14 wegbewegt werden. Vorzugsweise ist das Instrument 12 so ausgebildet, dass das zweite Werkzeugelement 16 von einer Gewebefassstellung, wie sie schematisch in den Figuren 2 und 3 dargestellt ist und in welcher die Werkzeugelemente 14, 16 einen maximalen Abstand 58 voneinander aufweisen, in die Annäherungs- oder Gewebeverbindungsstellung bringbar ist durch Verschwenken der Betätigungsglieder 78 aufeinander zu, was eine Bewegung des Kraftübertragungsglieds 80 in proximaler Richtung zur Folge hat.

Des Weiteren umfasst das Instrument 12 eine Schneideinrichtung 86 zum Durchtrennen von Gewebe. Die Schneideinrichtung umfasst ein Schneidelement 88 mit einer in sich geschlossenen ringförmigen Schneidkante 90. Die Schneidkante 90 definiert eine relativ zur Längsachse 54 des Instruments 12 geneigte Schneidebene 92. Die Schneidebene 92 ist um circa 10° bezogen auf eine senkrecht zur Längsachse 54 verlaufende Referenzebene geneigt, die parallel zu den Werkzeugelementflächen 32 und 33 verläuft. Proximalseitig ist am Schaft 24 ein weiterer HF-Schneidanschluss 94 vorgesehen, welcher bei einer Variante des Instruments 12 elektrisch leitend mit dem Schneidelement 88 verbunden ist. Damit könnte zum Beispiel eine monopolare Schneideinrichtung 86 realisiert werden, wobei zum monopolaren Schneiden am Körper des Patienten in üblicher Weise eine Neutralelektrode anzulegen wäre. Eine bipolare Schneideinrichtung 86 wird erfindungsgemäß dadurch verwirklicht, dass der Schneidkante 90 gegenüberliegend am zweiten Werkzeugelement 16 eine Ringelektrode 96 angeordnet ist, die über eine nicht näher dargestellte elektrisch leitende Verbindung, die beispielsweise in nicht dargestellter Weise durch das Kraftübertragungsglied 80 hindurch verläuft, mit einem weiteren HF-Schneidanschluss 94 verbunden ist. Die Ringelektrode 96 selbst kann wahlweise auch segmentiert sein, beispielsweise analog den HF-Elektroden 28 und 29. Es wäre auch möglich, statt der Ringelektrode 96 die HF-Elektrode 29 als Gegenelektrode zu nutzen.

Das Schneidelement 88 ist vorzugsweise relativ zu beiden Werkzeugelementen 14, 16 verschiebbar gelagert. Die konzentrisch um die Längsachse 54 ausgebildete Schneidkante 90 kann so relativ zu den HF-Elektroden 28 und 29 verschoben werden. Zum Betätigen der Schneideinrichtung 86 ist eine Schneidbetätigungseinrichtung 98 vorgesehen mit einem vom proximalen Ende des Instruments abstehenden Betätigungsglied 100. Dieses ist über einen nicht dargestellten Mechanismus, beispielsweise ein weiteres, im Inneren des Schafts 24 verlaufendes Kraftübertragungsglied, mechanisch mit dem Schneidelement 88 gekoppelt, so dass infolge einer Bewegung des Betätigungsglieds 100 auch das Schneidelement 88 bewegt wird. Vorzugsweise ist das Betätigungsglied 100 verschiebbar und rotierbar relativ zum Schaft 24 angeordnet, so dass das Schneidelement 88 nicht nur parallel zur Längsachse 54 verschoben, sondern auch relativ zu dieser verdreht werden kann.

Um die Elektrodensegmente 30, 31 in beliebiger Weise mit einem HF-Strom beaufschlagen zu können, ist eine Steuer- und/oder Regelungseinrichtung 102 mit einer Schalteinrichtung 104 vorgesehen. Die Steuer- und/oder Regelungseinrichtung 102 ist vorzugsweise in einem Gehäuse des HF-Stromgenerators 18 angeordnet und bildet einen Teil desselben. Die Schalteinrichtung 104 ist insbesondere ausgebildet zum sequentiellen Beaufschlagen der Elektrodensegmente 30, 31 mit einem HF-Strom. Die Schalteinrichtung 104 dient insbesondere zum Ansteuern der Kontakte 66 sowie weiterer Kontakte 106, die über weitere Verbindungsleitungen 108 mit den HF-Schneidanschlüssen 94 des Instruments 12 verbindbar sind. Auf diese Weise kann mit dem HF-Stromgenerator 18 die Schneideinrichtung 86 beispielsweise monopolar oder erfindungsgemäß bipolar betrieben werden. Zum monopolaren Betrieb wird lediglich das Schneidelement 88 mit einem HF-Strom beaufschlagt und als Gegenelektrode eine Neutralelektrode am Körper des Patienten angeordnet. Zum bipolaren Schneiden kann insbesondere eine ringförmige Gegenelektrode am zweiten Werkzeugelement 16 vorgesehen werden, zum Beispiel in Form der Ringelektrode 96, so dass dann ein HF-Strom zwischen der Gegenelektrode und dem Schneidelement 88 fließen kann. Alternativ kann auch die HF-Elektrode 29 als Gegenelektrode genutzt werden. Wird ganz auf eine Bestromung der Schneideinrichtung 86 verzichtet, so kann diese auch rein mechanisch zum Durchtrennen von Gewebe genutzt werden, und zwar mittels der vorzugsweise angeschliffenen Schneidkante 90.

Die Schalteinrichtung 104 kann ferner auch derart ausgebildet sein, dass mindestens zwei Elektrodensegmente 30, 31 einer HF-Elektrode 28, 29 gleichzeitig mit einem HF-Strom beaufschlagt werden können. Dabei ist es günstig, wenn jeweils zwischen zwei gleichzeitig mit HF-Strom beaufschlagten Elektrodensegmenten 30, 31 ein weiteres, jedoch dann unbestromtes Elektrodensegment 30, 31 angeordnet ist. Beispielsweise könnten auf diese Weise die einander gegenüberliegenden Elektrodensegmente 30 der in Figur 5 dargestellten HF-Elektrode 28 gleichzeitig bestromt werden, wobei die beiden anderen Elektrodensegemente 30 dann unbestromt blieben.

Um eine Bestromungsstärke und/oder eine Bestromungsdauer für die einzelnen Elektrodensegmente 30, 31 individuell einstellen zu können, ist die Steuer- und/oder Regelungseinrichtung 102 eine Einstelleinrichtung 110 umfassend ausgebildet. Mittels der Einstelleinrichtung 110 kann beispielsweise eine Stärke und/oder eine Frequenz des HF-Stroms eingestellt werden, ebenso wie eine Bestromungsdauer. Des Weiteren kann die Einstelleinrichtung 110 optional auch ausgebildet sein, um Bestromungssequenzen individuell einstellen zu können.

Des Weiteren umfasst die Steuer- und/oder Regelungseinrichtung 102 vorzugsweise eine Temperaturmesseinrichtung 112 zum Messen einer Elektrodensegmenttemperatur und/oder einer Gewebetemperatur. Die Temperaturmesseinrichtung 112 dient insbesondere dazu, der Steuer- und/oder Regelungseinrichtung 102 die für ein automatisches Regeln einer Bestromung der HF-Elektroden 28, 29 erforderliche Regelgröße zu liefern, nämlich eine Temperatur des Gewebes, beispielsweise indirekt über eine Temperaturmessung der Elektrodensegmente 30, 31. Beispielsweise können nicht bestromte Elektrodensegmente 30, 31 als Messkontakte zur Temperaturerfassung über eine Gewebeimpedanzmessung dienen. Auf diese Weise kann sicher gestellt werden, dass die zum Verbinden des Gewebes erforderliche Temperatur in gewünschter Weise und hochpräzise durch entsprechende Bestromung der HF-Elektroden 28, 29 erreicht wird, jedoch ein unerwünschtes Überhitzen der miteinander zu verbindenden Gewebeteile vermieden wird.

Ferner umfasst die Steuer- und/oder Regelungseinrichtung 102 optional eine Impedanzmesseinrichtung 113 zum Messen einer Gewebeimpedanz von zwischen den Werkzeugelementen 14 und 16 gehaltenem Gewebe. Die Bestimmung der Gewebeimpedanz bietet die Möglichkeit, abhängig von ihrem Wert den HF-Generator 18 zu regeln, insbesondere die von diesem bereitgestellten Parameter Spannung, Strom oder Leistung. Auf diese Weise kann die zum Verbinden der Gewebeteile in diese einzubringende Energie einfach und sicher geregelt werden. Zur Messung der Gewebeimpedanz können insbesondere die HF-Elektroden 28 und 29 genutzt werden. Eine Messung kann auch zwischen einzelnen Elektrodensegmenten 30 und 31erfolgen, die einander gegenüberliegen. Die Gewebeimpedanzmessung kann wahlweise während der Bestromung der HF-Elektroden 28, 29 stattfinden oder wenn die HF-Elektroden 28, 29 gerade unbestromt sind. Damit kann die Veränderung des Gewebes gut und praktisch in Echtzeit überwacht und weiterer Energieeintrag dosiert, unterbunden oder gezielt weiter gestattet werden.

Mit dem oben beschriebenen chirurgischen System 10 lassen sich insbesondere schlauchförmige Gewebeteile 116 direkt miteinander verbinden, indem diese durch HF-Strom-Beaufschlagung miteinander verschweißt oder versiegelt werden. Im Einzelnen wird dabei beispielsweise folgendermaßen vorgegangen:
Zum Herstellen einer End-zu-End-Anastomose von zwei schlauchförmigen Gewebeteilen 116, wie sie beispielsweise nach einer Darmoperation, bei welcher ein Stück Darm herausgetrennt wird, erforderlich ist, werden freie Enden der Gewebeteile 116 aneinander herangeführt, so dass sie mit ihren freien Enden in Richtung auf die Längsachse weisend, ringförmig flächig aneinander anliegen, wie beispielhaft in den Figuren 3 und 4 dargestellt. Die freien Enden befinden sich dann zwischen den beiden Werkzeugelementen 14, 16, so dass die Gewebeteile 116 klemmend zwischen den Werkzeugelementen 14, 16 in der Gewebefassstellung aneinander gehalten werden können.

Die Werkzeugelemente 14, 16 werden dann aufeinander zu bewegt in die Gewebeverbindungsstellung, so dass auch die Elektrodensegmente 31 elektrisch leitend mit den HF-Anschlusskontakten 50 in der oben beschriebenen Weise verbunden sind. Zum Verschweißen der Gewebeteile 116 werden nun vorzugsweise einzelne Elektrodensegmentpaare 62 mit einem HF-Strom beaufschlagt, der dann über die zwischen den Werkzeugelementen 14, 16 gehaltenen Gewebeteilabschnitte fließt und diese erwärmt. Bei einer Temperatur von etwa 50°C bis etwa 80°C, vorzugsweise circa 65°C bis 70°C erfolgt eine Veränderung in den Zellen derart, dass die Gewebeteile 116 miteinander verkleben. Das Verbindungsverfahren wird vorzugsweise derart durchgeführt, dass immer nur ein Elektrodensegmentpaar 62 gleichzeitig bestromt wird, insbesondere in einer sequentiellen Abfolge. Auf diese Weise wird eine ringförmige Verbindungslinie 114 hergestellt, welche im Wesentlichen durch die HF-Elektroden 28, 29 beziehungsweise deren Elektrodenmittellinien 44, 45 vorgegeben wird.

Dadurch, dass nicht die gesamten HF-Elektroden 28, 29 mit einem HF-Strom beaufschlagt werden, kann die Temperatur zum Verbinden der Gewebeteile 116 viel besser kontrolliert und eine Zerstörung der Zellen verhindert werden. Vorzugsweise werden die Elektrodensegmente 30, 31 nacheinander, also sequentiell bestromt, so dass die Gewebeteile 116 längs der Verbindungslinie 114 schrittweise miteinander verschweißt werden. Durch die zweireihige Anordnung der Elektrodensegmentabschnitte 38, 39, 40 und 41 wird ferner eine doppelte Verbindung zwischen den Gewebeteilen 116 hergestellt, die eine optimale Abdichtung und eine dauerhafte, stabile Verbindung der Gewebeteile 116 miteinander gewährleisten kann.

Alternativ zu einer sequentiellen Bestromung können, wie bereits oben angedeutet, auch gegenüberliegende Elektrodensegmente 30, 31 gleichzeitig bestromt werden, wodurch die Dauer zum Verbinden der Gewebeteile 116 bei dem in den Figuren 1 bis 5 schematisch dargestellten Ausführungsbeispiel halbiert werden kann.

Nach dem Verbinden der Gewebeteile 116 wird überstehendes Gewebe mittels der Schneideinrichtung 86 entfernt. Dabei wird die Schneideinrichtung 86 vorzugsweise bipolar genutzt, das heißt das Schneidelement 88 und die Ringelektrode 96 werden mit dem HF-Strom-Generator 18 verbunden und ein HF-Strom zum Durchtrennen des Gewebes über die beiden Gewebeteile 116 geleitet. Durch die geneigte Schneidkante 90 wird ein definierter Schneidfunke erzeugt, und zwar in dem Bereich, in dem der Abstand zwischen der Schneidkante 88 und der Ringelektrode 96 minimal ist. Ausgehend von diesem Bereich wandert dann der Schneidfunke automatisch entlang der Schneidkante 90 in beiden Richtungen im Kreis herum, bis das Gewebe vollständig durchtrennt ist. Die Nutzung der Schneideinrichtung 86 im bipolaren Betriebsmodus hat insbesondere den Vorteil, dass die Gewebeteile 116 beim Durchtrennen gleichzeitig auch koaguliert werden, um unerwünschte Blutungen direkt beim Durchtrennen zu stillen.

Nach dem Verbinden und Beschneiden der Gewebeteile 116 kann dann das Instrument 12 durch Zurückziehen des Schafts 24 aus dem Körper des Patienten, beispielsweise aus dessen Darm, zurückgezogen werden.

Der Schaft 24 ist, je nach Ausgestaltung des Instruments 12, vorzugweise so lang, dass beim Einsatz des Instruments 12 sowohl die Betätigungseinrichtung 76 als auch die Schneidbetätigungseinrichtung 98 noch aus dem Körper des Patienten herausragen, so dass sie von einem Operateur betätigt werden können.

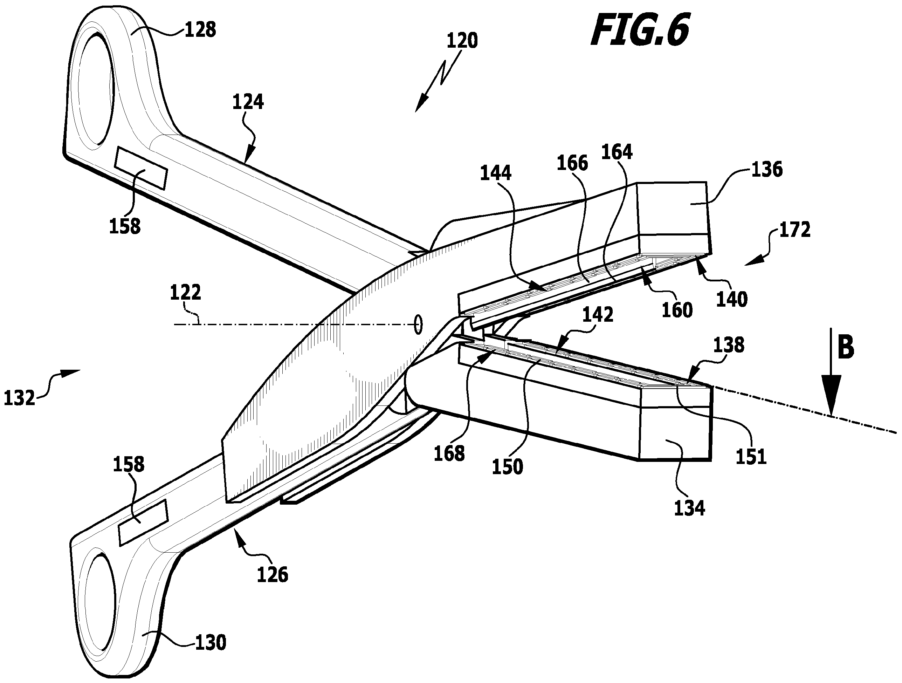

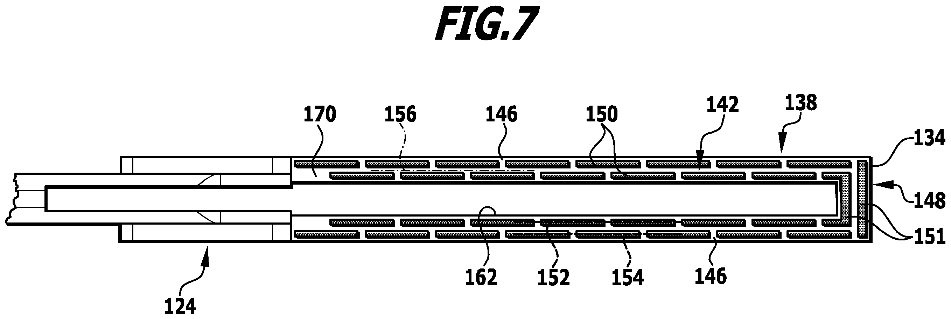

Alternativ oder zusätzlich kann das chirurgische System 10 anstelle des Instruments 12 auch ein chirurgisches Instrument beispielsweise in Form eines schematisch in den Figuren 6 und 7 dargestellten Instruments 120 umfassen. Das Instrument 120 umfasst zwei relativ zueinander um eine Schwenkachse 122 verschwenkbar aneinander gelagerte Branchen 124 und 126. An einem proximalen Ende der Branchen 124, 126 sind Fingerringe 128, 130 ausgebildet, die zusammen eine Betätigungseinrichtung 132 definieren zum Betätigen des Instruments 120.

Ausgehend von freien, distalen Enden 134 und 136 der Branchen 124 und 126 sind aufeinander zuweisend an Innenseiten derselben Werkzeugelemente 138 und 140 ausgebildet. Die Werkzeugelemente 138 und 140 sind identisch ausgebildet und liegen in einer Annäherungsstellung der Enden 134 und 136 einander gegenüber und weisen in dieser Stellung einen minimalen Abstand voneinander auf. Jedes Werkzeugelement 138, 140 umfasst eine HF-Elektrode 142, 144, die identisch und im Wesentlichen U-förmig ausgebildet sind. Jede HF-Elektrode 142, 144 umfasst zwei parallel zueinander verlaufende, sich in einer Richtung senkrecht zur Schwenkachse 122 erstreckende Elektrodenabschnitte 146 sowie einen senkrecht zu diesen verlaufenden, an den Enden 134, 136 angrenzenden Elektrodenabschnitt 148.

Der Aufbau der HF-Elektroden 142, 144 wird nachfolgend beispielhaft in Verbindung mit Figur 7 anhand der HF-Elektrode 142 näher beschrieben.

Die HF-Elektrode 142 umfasst insgesamt 30 Elektrodensegmente 150, wobei jeweils 15 Elektrodensegmente versetzt zueinander in zwei Elektrodenreihen 152, 154 parallel zueinander längs jedes Elektrodenabschnitts 146 angeordnet und gegeneinander elektrisch isoliert sind. Die Elektrodensegmente 150 sind geradlinig und streifenförmig ausgebildet. Sie definieren zwischen sich eine Elektrodenmittellinie 156, die der Form der HF-Elektrode 142 entsprechend ebenfalls U-förmig ausgebildet ist. Im Bereich des Elektrodenabschnitts 148 sind zwei weitere Elektrodensegmente 151 angeordnet, die jeweils die Elektrodenreihen 152 beziehungsweise 154 der Elektrodenabschnitte 146 vervollständigen. Die Elektrodensegmente 150 und 151 sind somit in einer von der Elektrodenmittellinie 156 definierten Richtung versetzt zueinander angeordnet.

Um die Elektrodensegmente 150, 151 mit einem HF-Strom beaufschlagen zu können, sind diese jeweils elektrisch leitend mit einem HF-Anschluss 158 in proximalen Endbereichen der Branchen 124, 126 benachbart der Fingerringe 128, 130 angeordnet. Die HF-Anschlüsse 158 können mit entsprechenden Verbindungsleitungen oder Kabeln mit dem HF-Stromgenerator 18 verbunden werden.

In der Annäherungsstellung liegen aufgrund der identischen Ausbildung der HF-Elektroden 142 und 144 gleich große oder im Wesentlichen gleich große Elektrodensegmente 150 beziehungsweise 151 einander gegenüber und weisen aufeinander zu. Sie bilden ein insgesamt mit dem Bezugszeichen 168 bezeichnetes Elektrodensegmentpaar. Insgesamt umfasst das Instrument 120 somit 32 Elektrodensegmentpaare 168.

Auch die Werkzeugelemente 138 und 140 definieren ebene Werkzeugelementflächen 170, die U-förmig ausgebildet sind. Die Elektrodensegmente 150 und 151 stehen nicht über die Werkzeugelementfläche 170 vor.

Das insgesamt zangenförmig ausgebildete Instrument 120 kann ebenfalls zum Verbinden von Gewebeteilen genutzt werden, wobei diese zwischen den Werkzeugelementen 138, 140 klemmend gehalten und dann durch entsprechende Strombeaufschlagung der Elektrodensegmente 150, 151 miteinander verschweißt oder versiegelt werden. Dazu kann, wie im Zusammenhang mit der Funktion des Instruments 12 beschrieben, eine Bestromung der Elektrodensegmente 150 sequentiell erfolgen, das heißt U-förmig umlaufend wird nach Bestromen eines Elektrodensegments 150 das nächstgelegene Elektrodensegment 150 der benachbarten Elektrodenreihe 152, 154 bestromt, bis alle Elektrodensegmente 150, 151 einmal bestromt waren. Auf diese Weise kann eine zweireihige Verbindungslinie zum Verbinden zweier Gewebeteile hergestellt werden. Alternativ ist auch beim Instrument 120 eine gleichzeitige Bestromung von zwei oder auch mehr Elektrodensegmenten 150, 151 denkbar, wobei vorzugsweise aneinander angrenzende Elektrodensegmente 150, 151 nicht gleichzeitig bestromt werden, sondern bevorzugt mindestens eines, besser zwei oder drei Elektrodensegmente 150, 151 zwischen gleichzeitig bestromten Elektrodensegmenten 150, 151 unbestromt bleiben.

Das Instrument 120 kann optional auch eine Schneideinrichtung 160 umfassen, wie sie schematisch in Figur 6 dargestellt ist. Zwischen den Elektrodenabschnitten 146 ist jeweils ein Schlitz 162 an den Werkzeugelementen 138, 140 ausgebildet. Im Schlitz 162 an der Branche 126 ist ein Schneidelement 164 mit einer in Richtung auf den Schlitz 162 der Branche 124 weisenden Schneidkante 166 gehalten und optional relativ zum Werkzeugelement 136 bewegbar. Damit kann zum Beispiel bereits beim Schließen der Branchen 124 und 126 das zwischen den Werkzeugelementen 138 und 140 gehaltene Gewebe durchtrennt werden. Optional kann das Schneidelement 164 auch monopolar oder bipolar genutzt werden, wobei beispielsweise die HF-Elektrode 142 als Gegenelektrode zum Schneidelement 164 bei einem biopolaren Einsatz genutzt werden kann. Zum monopolaren Betrieb wird lediglich das Schneidelement 164 mit einem HF-Strom beaufschlagt und als Gegenelektrode eine Neutralelektrode am Körper des Patienten angeordnet. In beiden Fällen ist das Schneidelement 164 vorzugsweise auch elektrisch leitend mit einem Kontakt der HF-Anschlüsse 158 verbunden.

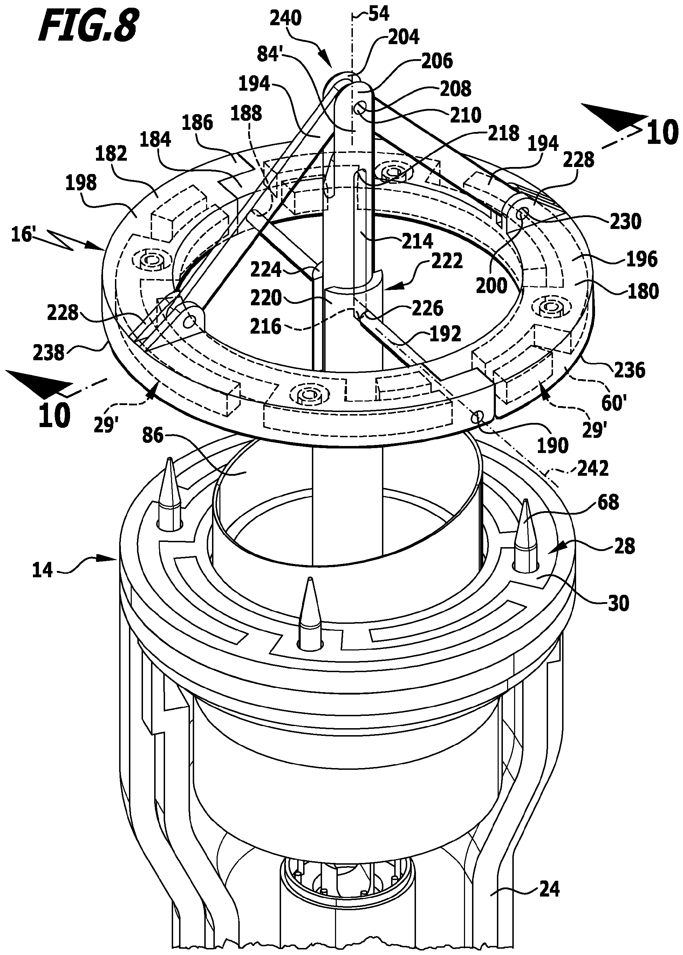

In den Figuren 8 bis 11 ist eine Variante des Instruments 12 dargestellt, das sich durch die Ausgestaltung des zweiten Werkzeugelements unterscheidet, das in den Figuren 8 bis 11 mit dem Bezugszeichen 16' bezeichnet ist. Das Werkzeugelement 16' nimmt in einer Betriebsstellung, in welcher es in die oben beschriebene Annäherungsstellung bringbar ist, eine kreisringfähige Form ein. Es umfasst zwei Kreisringabschnitte 180 und 182, die sich jeweils über einen auf die Längsachse 54 bezogenen Winkel von etwa 180° erstrecken. Freie Enden der Kreisringabschnitte 180, 182 sind nur halb so breit wie die Kreisringabschnitte 180, 182 im übrigen Bereich und dienen als Lagerböcke 184 und 186. Die Lagerböcke 184 und 186 sind jeweils mit einer Querbohrung 188 und 190 versehen, in die ein zylindrischer Stab 192 eingesetzt ist. Die Lagerböcke 184 liegen an den Lagerböcken 186 auf deren der Längsachse 54 zugewandten Seite an. Der Stab 192 ist drehfest in den Querbohrungen 190 des Kreisringabschnitts 182 festgelegt. Die Querbohrung 188 ist in ihrem Innendurchmesser so bemessen, dass der Kreisringabschnitt 180 relativ zum Stab 192 um eine von diesem definierte Schwenkachse 242 und damit relativ zum Kreisringabschnitt 182 verschwenkbar ist.

Die beiden Kreisringabschnitte 180 und 182 sind jeweils zusätzlich über einen stabförmigen Lenker 194 mit einem Halteglied 84', welches eine mit der Längsachse 54 zusammen fallende Haltegliedlängsachse definiert, gekoppelt. Das Halteglied 84' ist, analog wie das Halteglied 84, mit dem Kraftübertragungsglied 80 gekoppelt beziehungsweise koppelbar, und auf diese Weise relativ zum Schaft 24 in distaler und proximaler Richtung bewegbar. Zum beweglichen Anlenken der Lenker 194 am Halteglied 84' ist letzteres im Bereich seines distalen Endes mit einem Schlitz 204 versehen, welcher sich quer zu einer vom Stab 192 definierten Längsachse erstreckt. Auf diese Weise werden zwei Schenkel 206 ausgebildet, die mit einer fluchtenden Querbohrung 208 versehen sind, in welche ein zylindrischer Lagerstift 210 drehfest eingesetzt ist. Die Lenker 194 sind an ihren ersten Enden mit einer Aufnahmebohrung 212 versehen, durch die sich der Lagerstift 210 erstreckt und die einen Innendurchmesser aufweist, um eine Verschwenkbewegung der Lenker 194 um eine vom Lagerstift 210 definierte Schwenkachse zu gestatten.

Etwas proximalseitig des Schlitzes 204 erstreckt sich im Halteglied 84' weiter in proximaler Richtung ein Längsschlitz oder Langloch 214, welches vom Stab 192 durchsetzt ist. Auf diese Weise ist der Stab 192 definiert und parallel zu sich selbst in einer Richtung parallel zur Längsachse 54 verschiebbar. Ein proximales Ende des Langlochs 214 bildet einen proximalen Endanschlag für den Stab 192, ein distales Ende 218 des Langlochs 214 einen distalen Endanschlag für den Stab 192.

Zum Bewegen des Stabes 192 dient ein Betätigungsmechanismus 222, welcher ein hülsenförmiges Kraftübertragungselement 220 umfasst, dessen Innendurchmesser an den Außendurchmesser des Halteglieds 84' angepasst und somit auf dem Halteglied 84' in distaler und proximaler Richtung verschiebbar ist. Das Kraftübertragungselement 220 ist benachbart seines distalen Endes 224 mit einer Bohrung 226 versehen, die der Stab 192 durchsetzt. Der Stab 192 ist relativ zur Bohrung 226 rotierbar. Der Betätigungsmechanismus 222 kann ferner einen Teil des oben beschriebenen Betätigungsmechanismus 76 bilden. Dies bedeutet, dass eine Bewegung des Stabes 192 beispielsweise auch durch eine Verschwenkung der Betätigungsglieder 100 relativ zueinander möglich ist. Alternativ wäre es denkbar, eine weitere Betätigungseinrichtung analog der Betätigungsmechanismus 76 vorzusehen, die ein oder zwei weitere Betätigungsglieder, ähnlich den Betätigungsgliedern 100, umfasst, um gezielt eine Relativbewegung zwischen dem Kraftübertragungselement 220 und dem Halteglied 84' zu realisieren.

Auf Oberseiten der Kreisringabschnitte 180 und 182 sind jeweils parallel zueinander zwei Lagerböcke 228 angeordnet, die parallel zur Querbohrung 208 mit Bohrungen 230 versehen sind. Zwischen den Lagerböcken 228 ist jeweils ein weiteres freies Ende der Lenker 194 auf der in den Bohrungen 230 eingesetzten Lagerwelle 200 verschwenkbar gelagert. Durch die beschriebene Anordnung der Lenker 194, die auch als Anlenkglieder bezeichnet werden können, ist sichergestellt, dass sie mit einem Ende am zweiten Werkzeugelement 16' an einem Angriffs- oder Gelenkpunkt angreifen, welcher von der Schwenkachse 242 beabstandet ist.

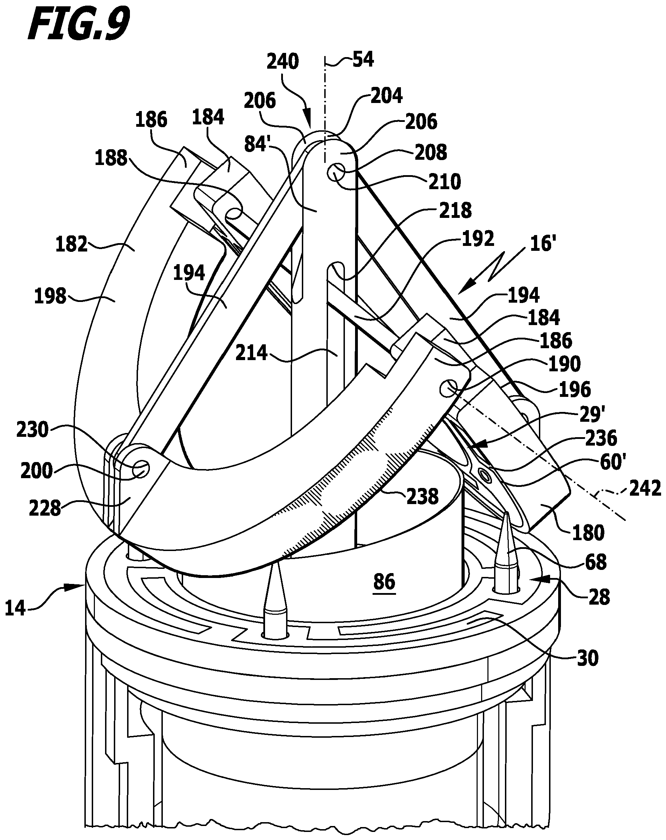

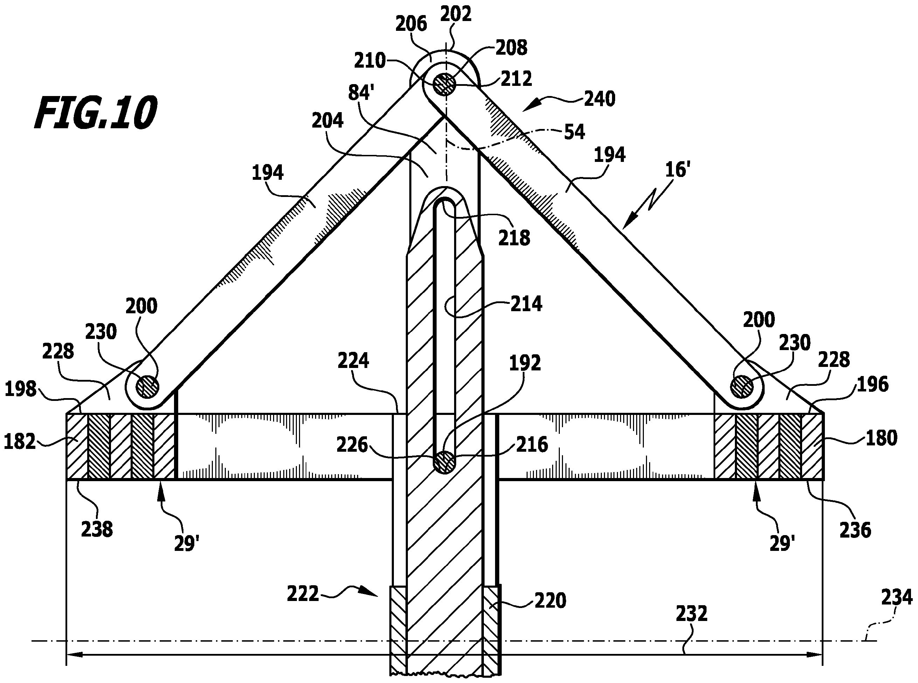

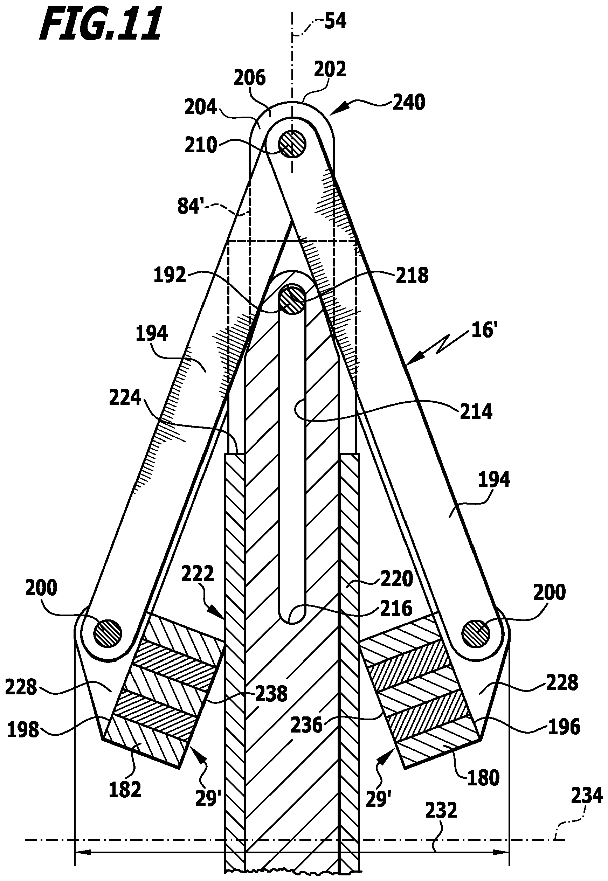

Mittels des Betätigungsmechanismus 222 kann das zweite Werkzeugelement 16' von der bereits erwähnten Betriebsstellung, die schematisch in den Figuren 8 und 10 dargestellt ist, in die Entfernungsstellung gebracht werden, die beispielhaft in Figur 11 dargestellt ist. Figur 9 stellt eine Zwischenstellung schematisch dar, also eine Stellung, zwischen der Betriebsstellung und der Entfernungsstellung. Wie durch Vergleich der beiden Figuren 10 und 11 leicht erkennbar, ist ein Flächenbereich einer senkrechten Projektion des zweiten Werkzeugelements 16' auf eine Projektionsebene 234, welche senkrecht zur Längsachse 54, also zur Schaftrichtung im Bereich des zweiten Werkzeugelements 16', verläuft, in der Entfernungsstellung kleiner ist als in der Betriebsstellung. Dies wird durch eine Bewegung des hülsenförmigen Kraftübertragungselements 220 ausgehend von der Betriebsstellung erreicht, in der der Stab 192 am proximalen Ende 216 anschlägt und sich Unterseiten 236 und 238 der Kreisringabschnitte 180 und 182 parallel zur Projektionsebene 234 erstrecken. Bewegt man das Kraftübertragungselement 220 in distaler Richtung, wird der Stab 192 im Langloch 214 in distaler Richtung zwangsgeführt. Durch die gelenkige Verbindung der Kreisringabschnitte 180 und 182 relativ zueinander und über die beiden Lenker 194 mit dem Halteglied 84' schwenken die Kreisringabschnitte 180 und 182 um die Schwenkachse 242 in Richtung auf die Längsachse 54 hin. Das zweite Werkzeugelement 16' wird auf diese Weise zusammengefaltet oder zusammengeklappt. Es ist also durch die gelenkige Anordnung der Kreisringabschnitte 180 und 182 mittels der Lenker 194 ein Faltmechanismus 240 ausgebildet zum Überführen des zweiten Werkzeugelements 16' von der Betriebsstellung in die Entfernungsstellung.

Bisher nicht erwähnt ist die Ausgestaltung der Unterseiten 236 und 238 des zweiten Werkzeugelements. Diese können entweder eine einzige, im Wesentlichen durchgehende Ringelektrode aufweisen, die eine einzige Gegenelektrode zur HF-Elektrode 28 des ersten Werkzeugelements 14 bildet. Alternativ kann auf den Unterseiten 236 und 238 analog der HF-Elektrode 29 auch eine HF-Elektrode mit zwei oder mehr Elektrodensegmenten 31 ausgebildet sein, vorzugsweise entsprechend der HF-Elektrode 29. Dies gestattet dann in der Betriebsstellung ein Verbinden von Gewebeteilen 116 in der oben beschriebenen Weise.

Nach dem Verbinden der Gewebeteile kann dann der Faltmechanismus 240 betätigt werden, beispielsweise durch entsprechende Betätigung des beschriebenen Betätigungsmechanismus 222, wodurch das Halteglied 84' in distaler Richtung bewegt wird. Ist das Kraftübertragungselement 220 beispielsweise unbeweglich relativ zum Schaft 24 angeordnet, dann kann bei einer Bewegung in distaler Richtung des Kraftübertragungsglieds 80 automatisch das zweite Werkzeugelement 16' zusammengeklappt werden. Durch den in der Entfernungsstellung deutlich herabgesetzten Flächenbedarf kann das zweite Werkzeugelement durch eine durch das Verbinden der Gewebeteile 116 ausgebildete Verbindungsstelle hindurchgeführt werden beim Entfernen des Instruments 12, und zwar ohne die Verbindungsstelle zu dehnen, was deutlich schonender ist, als das zweite Werkzeugelement in der Betriebsstellung durch die Verbindungsstelle hindurchzuführen.

Es versteht sich von selbst, dass elektrisch leitende Verbindungen von der Elektrode 29 zu den HF-Anschlusskontakten 50 zum Beispiel über die Lenker 94 und das Halteglied 84' zu den HF-Anschlusskontakten 50 im proximalen Endbereich des Schafts 24 geführt werden können.

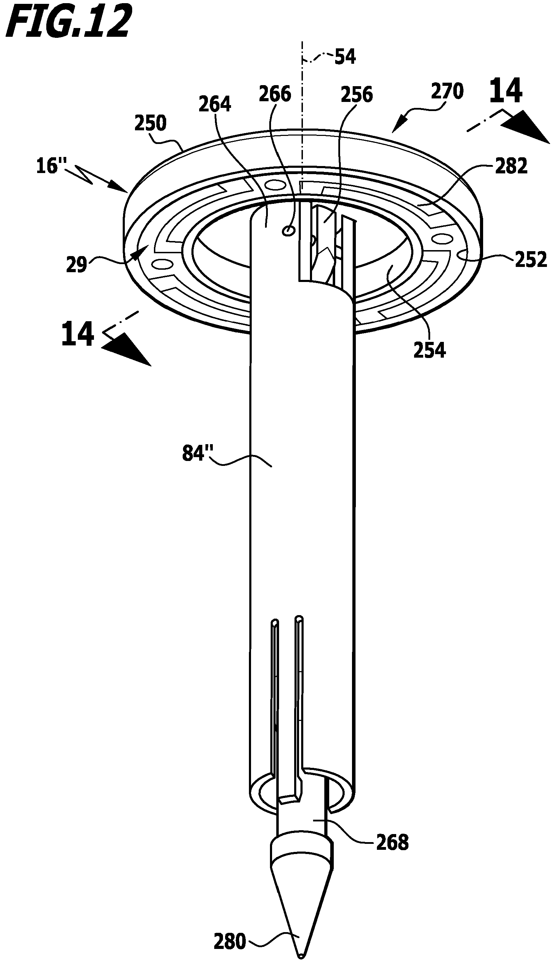

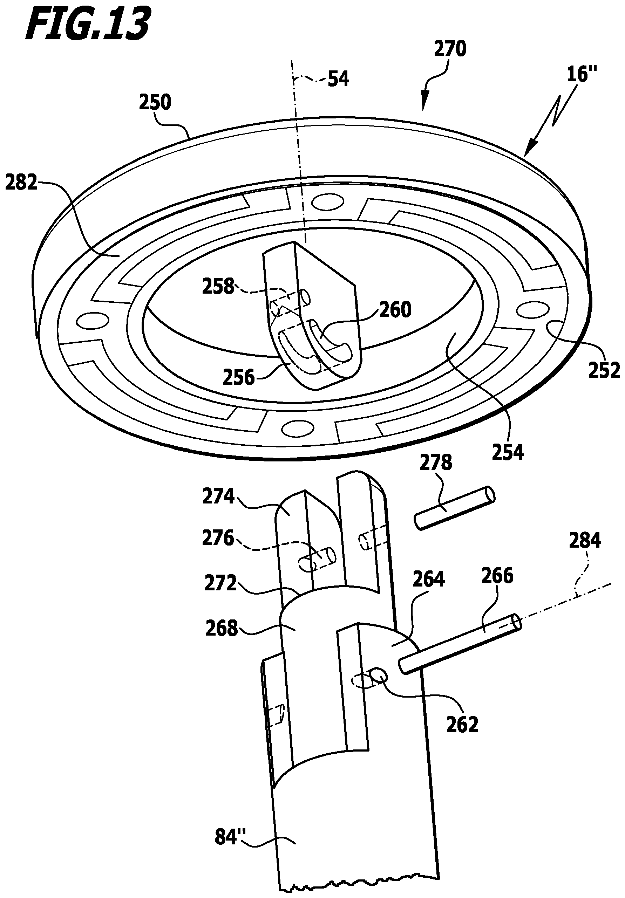

Eine weitere Variante eines zweiten Werkzeugelements ist in den Figuren 12 bis 15 insgesamt mit dem Bezugszeichen 16" bezeichnet. Es ersetzt beispielsweise die oben beschriebenen Werkzeugelemente 16 und 16' des Instruments 12.

Das zweite Werkzeugelement 16" ist im Wesentlichen tellerförmig ausgebildet mit einer in distaler Richtung weisenden, schwach konvex gekrümmten Außenseite 250.

Auf einer Unterseite des zweiten Werkzeugelements 16" ist eine Ringnut 252 ausgebildet, die in proximaler Richtung weisend geöffnet ist. Zentralmittig ist eine im Wesentlichen kreisförmige Vertiefung 254 ausgebildet, in der ein im Wesentlichen quaderförmiger Lagervorsprung 256 angeordnet ist, welcher koaxial zur Längsachse 54 in proximaler Richtung von der Unterseite des zweiten Werkzeugelements 16" abstehend ausgebildet ist. Der Lagervorsprung 256 ist mit einer Querbohrung 258 versehen, welche bezogen auf die Längsachse 54 windschief verläuft. Des Weiteren ist am Lagervorsprung 256 ein gekrümmter Führungsschlitz 260 ausgebildet, welcher in proximaler Richtung weisend konvex gekrümmt ist. Ein proximales Ende des Lagervorsprungs 256 weist eine abgerundete Außenkontur auf.

Das zweite Werkzeugelement 16" ist verschwenkbar an einem hülsenförmigen Halteglied 84" gelagert. Zu diesem Zweck ist das Halteglied 84" mit einer Querbohrung 262 versehen, die eine Wand 264 des Halteglieds 84" an zwei Stellen durchsetzt. In die Querbohrung 262 ist ein Lagerstift 266 drehfest eingesetzt. Er durchsetzt gleichzeitig die Querbohrung 258 derart, dass der Lagervorsprung 256 um eine vom Lagerstift 266 definierte Schwenkachse 284 verschwenkbar ist. Um einen auch beim zweiten Werkzeugelement 16" vorgesehenen Faltmechanismus 270 betätigen zu können, ist ein Kraftübertragungselement 268 vorgesehen, welches im Wesentlichen stabförmig ausgebildet ist und das Halteglied 84" koaxial zur Längsachse 54 durchsetzt. Von einer distalseitigen Endfläche 272 des Kraftübertragungselements 268 sind zwei Lagerschenkel 274 parallel zueinander und in distaler Richtung weisend abstehend angeordnet, welche jeweils von einer fluchtenden Bohrung 276 durchsetzt sind. In die Bohrungen 276 drehfest eingesetzt ist ein weiterer Lagerstift 278, welcher parallel zum Lagerstift 266 orientiert ist. Ein Außendurchmesser des Lagerstifts 278 ist so bemessen, dass er den Führungsschlitz 260 durchsetzen und relativ zu diesem bewegt werden kann.

Ein proximales Ende 280 des Kraftübertragungselements 268 ist vorzugsweise mit dem Kraftübertragungsglied 80 koppelbar, so dass sich in Folge einer Bewegung desselben auch das zweite Werkzeugelement 16" bewegen lässt.

In die Ringnut 252 eingesetzt ist ein ringförmiges Elektrodenelement 282, welches vorzugsweise eine HF-Elektrode 29 in der oben beschriebenen Form umfasst, die in den Figuren 12 bis 15 der Übersichtlichkeit wegen nicht im Einzelnen dargestellt ist. Alternativ kann auch eine einfache, durchgehende Ringelektrode am Elektrodenelement 282 ausgebildet sein.

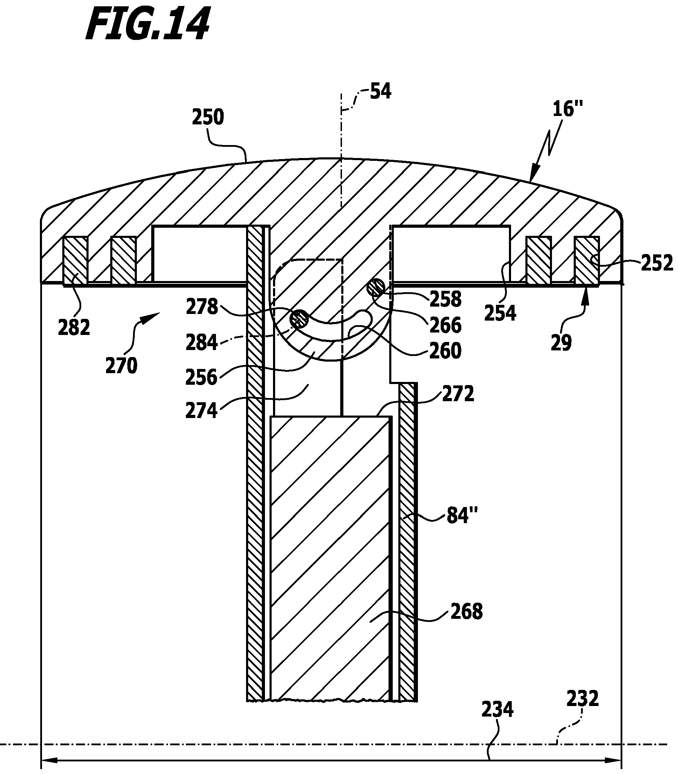

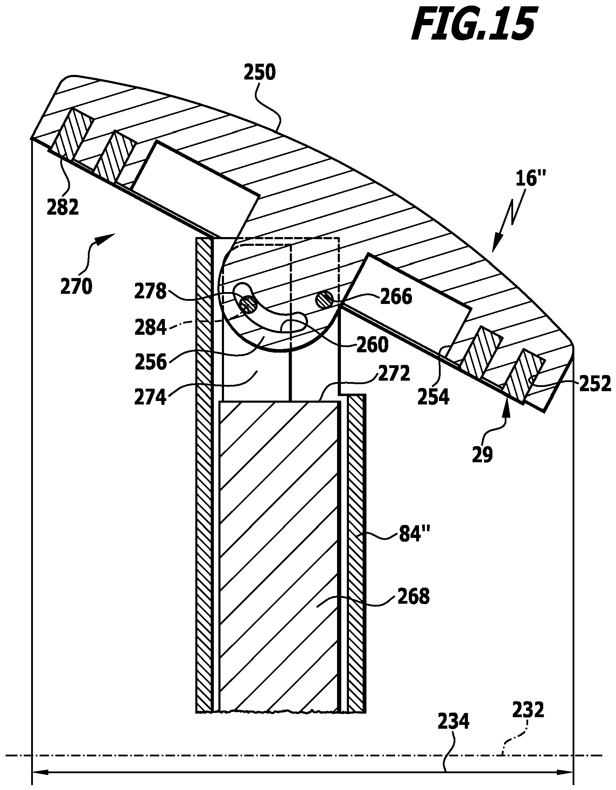

Zum Überführen des zweiten Werkzeugelements 16" von der Betriebsstellung in die Entfernungsstellung wird das Kraftübertragungselement 268 in distaler Richtung bewegt. Durch den speziell gekrümmten Führungsschlitz 260 wird der Lagerstift 278 in diesem zwangsgeführt und bewirkt somit eine zwangsgeführte Verschenkung des zweiten Werkzeugelements 16" um die Schwenkachse 284. Im Wesentlichen kann so das zweite Werkzeugelement 16" um nahezu 90° verschwenkt werden, so dass auch bei dieser Variante des Werkzeugelements 16" eine senkrechte Projektion 232 desselben auf die Projektionsebene 234 in der Entfernungsstellung kleiner ist als in der Betriebsstellung, wie dies schematisch in den Figuren 14 und 15 dargestellt ist. Auf diese Weise wird in der Entfernungsstellung beim Entfernen des Instruments 12 eine Überdehnung der Verbindungsstelle zwischen den miteinander verbundenen Gewebeteilen 116 vermieden.

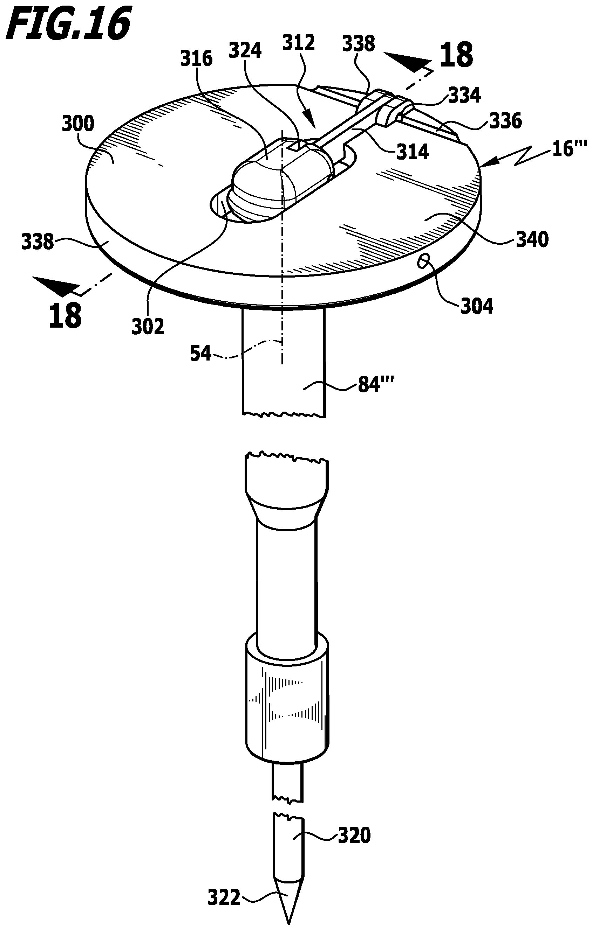

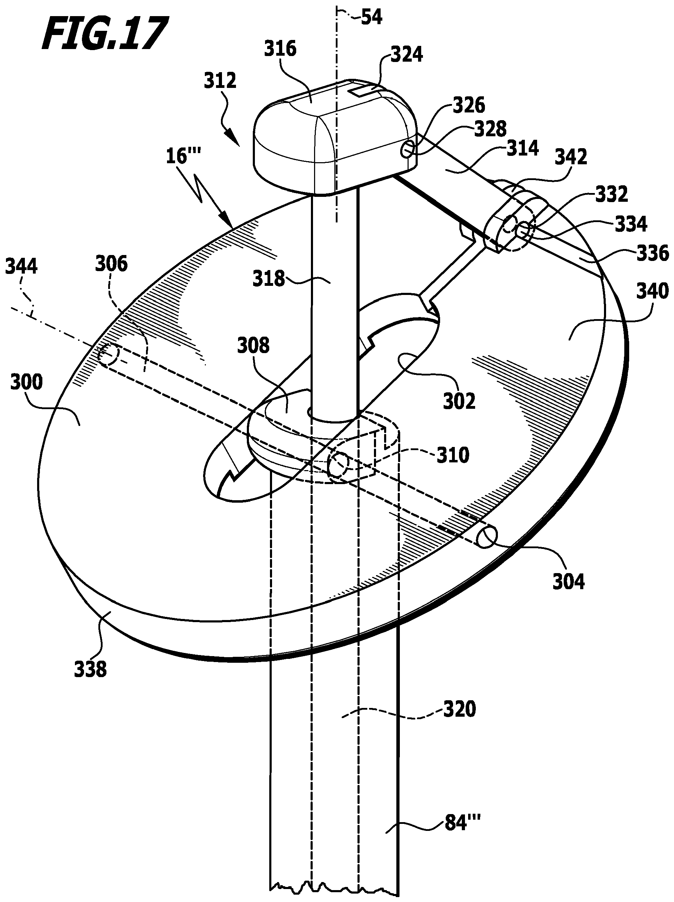

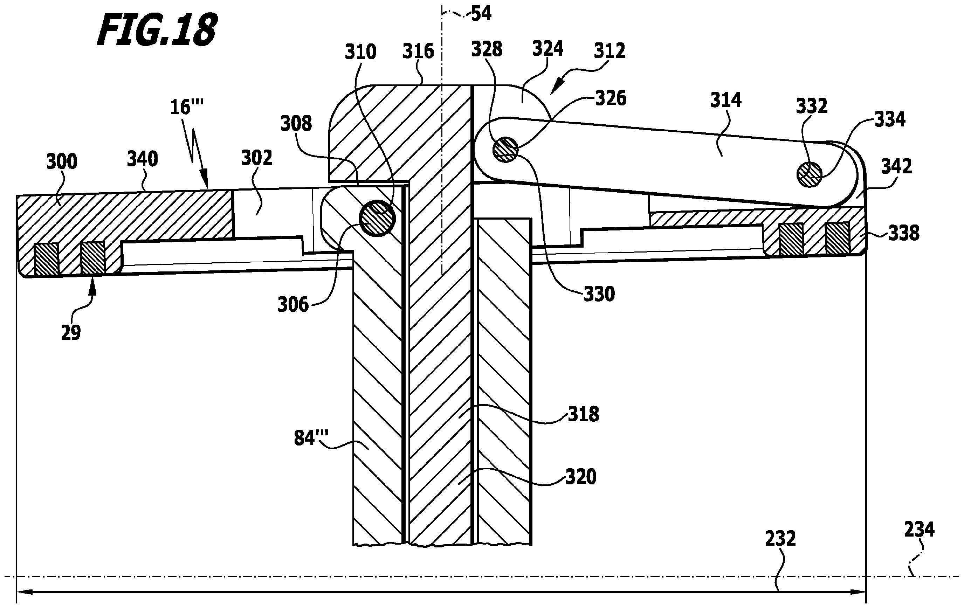

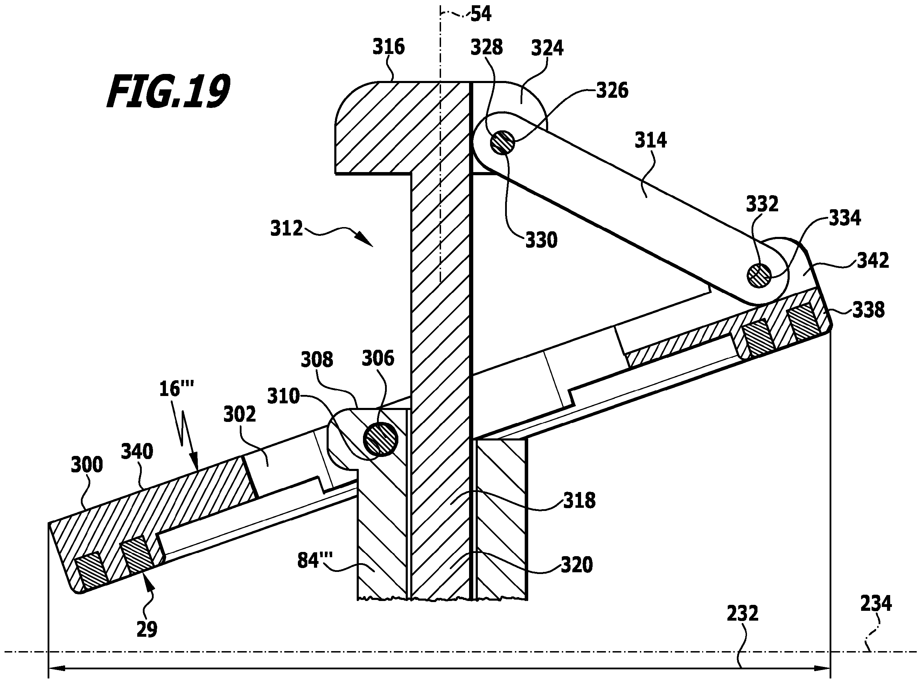

Eine weitere Ausführungsform eines insgesamt mit dem Bezugszeichen 16'" versehenen zweiten Werkzeugelements ist in den Figuren 16 bis 19 dargestellt. Es kann beim Instrument 12 anstelle der bisher beschriebenen zweiten Werkzeugelemente 16, 16' und 16" genutzt werden.

Das zweite Werkzeugelement 16'" ist im Wesentlichen tellerförmig ausgebildet und umfasst eine Scheibe 300. Diese ist in ihrem Zentrum mit einem quer verlaufenden, länglich ovalen Schlitz 302 versehen. Eine Bohrung 304 durchsetzt die Scheibe 300 etwas seitlich versetzt zu deren Mittelpunkt, der im Bereich des Schlitzes 302 liegt. In die Bohrung 304 drehfest eingesetzt ist ein Lagerstift 306, der den Schlitz 302 ebenfalls durchsetzt. In den Bereich des Schlitzes 302 hinein ragt ein distales Ende eines Halteglieds 84"', welches hülsenförmig ausgebildet ist. Das Halteglied 84'" ist proximalseitig von seinem Ende 308 mit einer Bohrung 310 versehen, deren Innendurchmesser an den Außendurchmesser des Lagerstifts 306 derart angepasst ist, dass der Lagerstift 306 relativ zur Bohrung 310 in dieser rotierbar ist. Dies ermöglicht dann insgesamt eine Verschwenkung der Scheibe 300 um eine vom Lagerstift 306 definierte Längsachse.

Zum zwangsbetätigten Verschwenken der Scheibe 300 dient ein Faltmechanismus 312, welcher die Scheibe 300 über einen Lenker 314 gelenkig mit einem distalen Ende 316 eines Kraftübertragungselements 318 koppelt. Das Kraftübertragungselement 318 weist einen langgestreckten, stabförmigen Abschnitt 320 auf, dessen proximales Ende 322 mit dem Kraftübertragungsglied 80 koppelbar ist. Das Ende 316 ist gegenüber dem Abschnitt 320 kopfförmig verdickt und nahezu quaderförmig geformt. An einer Seite desselben ist ein seitlich geöffneter Schlitz 324 ausgebildet. Ferner ist eine Querbohrung 326 vorgesehen, die den Schlitz 324 quer durchsetzt. In die Querbohrung 326 drehfest eingesetzt ist ein Lagerstift 328. Der stabförmige Lenker 314 ist ebenfalls mit einer Bohrung 330 versehen und auf dem Lagerstift 328 verschwenkbar gelagert. Benachbart eines gegenüber liegenden Endes des Lenkers 314 ist eine weitere Bohrung 332 vorgesehen. Sie dient zur Lagerung des Lenkers 314 auf einem weiteren Lagerstift 334. Dieser ist in einer weiteren Bohrung 336 der Scheibe 300 eingesetzt. Die Bohrung 336 ist parallel zur Bohrung 304 orientiert und außerhalb des Schlitzes 302 benachbart eines Randes 338 der Scheibe 300 angeordnet, und zwar der Bohrung 304 bezogen auf die Längsachse 54 gegenüberliegend. Auf einer Oberseite 340 der Scheibe 300 ist ausgehend vom Rand 338 eine Nut 342 eingearbeitet, in die das Ende des Lenkers 314 mit dessen Bohrung 332 eintaucht. Auf diese Weise ist der Lenker 314 gelenkig auf dem Lagerstift 334 gelagert. Damit greift der Lenker 314 mit einem Ende am zweiten Werkzeugelement 16'" an einem Angriffs- oder Gelenkpunkt an, welcher von einer von der Längsachse des Lagerstifts 306 definierten Schwenkachse 344 beabstandet ist.

Der Faltmechanismus 312 wird betätigt, indem das Kraftübertragungselement 318 in distaler Richtung bewegt wird. Dies hat zur Folge, dass der Lenker 314 relativ zur Scheibe 300 abgewinkelt wird. Je weiter das Kraftübertragungsglied 318 in distaler Richtung bewegt wird, umso weiter zieht dabei der Lenker 314 den Bereich der Scheibe 300 in distaler Richtung, an welchem die Nut 342 vorgesehen ist. In einer extremen Stellung ist dann die Scheibe 300 nahezu parallel zur Längsachse 54 ausgerichtet. Insgesamt ist es somit auch beim zweiten Werkzeugelement 16'" möglich, eine Entfernungsstellung so zu realisieren, in welcher eine senkrechte Projektion 232 desselben auf die Projektionsebene 234, welche senkrecht zur Längsachse 54 verläuft, kleiner ist als in der Betriebsstellung.

Am zweiten Werkzeugelement 16'" kann ebenfalls eine HF-Elektrode 29 in einer Form wie oben beim zweiten Werkzeugelement 16 beschrieben angeordnet oder ausgebildet sein. Alternativ ist es auch denkbar, eine in sich geschlossene, ringförmige Elektrode vorzusehen, die nicht in Elektrodensegmente unterteilt ist. Ähnlich wie das zweite Werkzeugelement 16" das Elektrodenelement 282 umfasst, können bei den zweiten Werkzeugelementen 16' und 16'" ebenfalls Elektrodenelemente vorgesehen sein, beispielsweise in Form des Elektrodenelements 282 oder aber auch des Elektrodenelements 52.

Wie bereits oben im Zusammenhang mit dem zweiten Werkzeugelement 16' erwähnt, können die an den zweiten Werkzeugelementen 16" und 16'" vorgesehenen HF-Elektroden in üblicher Weise durch Vorsehen entsprechender elektrisch leitender Verbindungen am Instrument 12 mit den HF-Anschlusskontakten 50 verbunden werden.

Alle oben beschriebenen ersten und zweiten Werkzeugelemente 14, 16, 16', 16", 16'" sowie 138 und 140 sind vorzugsweise aus entweder elektrisch leitenden oder elektrisch isolierenden Bauteilen aufgebaut. Denkbar sind auch Bauteile, die teilweise elektrisch leitend und teilweise elektrisch isolierend sind. Die Bauteile selbst können insbesondere komplett aus elektrisch leitenden oder elektrisch isolierenden Materialien hergestellt sein, wobei die elektrisch isolierenden Bauteile auch aus einem elektrisch leitenden Material hergestellt sein können, das insbesondere mit einer elektrisch isolierenden äußeren Hülle oder Beschichtung versehen ist. Als elektrisch isolierende oder nicht leitende Materialien können insbesondere Kunststoffe verwendet werden, welche bei den im Einsatz des chirurgischen Systems 10 auftretenden Temperaturen noch eine ausreichende Festigkeit aufweisen. Es eignen sich zum Beispiel sowohl Thermoplaste als auch Duroplaste. Alternativ kann als Isoliermaterial auch keramisches Material zum Einsatz kommen. Insbesondere können die Bauteile der Werkzeugelemente 14, 16, 16', 16", 16'" sowie 138 und 140 aus einer Keramik hergestellt sein. Eine Keramik zu verwenden hat insbesondere gegenüber vielen Kunststoffen den Vorteil, dass sie auch bei sehr hohen Temperaturen eine ausreichende Stabilität aufweist. Die HF-Elektroden 28 und 29 sind vorzugsweise aus einem Metall oder einer Metalllegierung hergestellt. Alternativ ist auch der Einsatz von elektrisch leitenden Keramiken zur Ausbildung der HF-Elektroden 28 und 29 denkbar, sofern sie die Anforderungen der Anwendung von HF-Strom erfüllen.

Die Werkzeugelemente 14, 16, 16', 16", 16'" sowie 138 und 140 können beispielsweise wie nachfolgend beschrieben hergestellt werden. Die einzelnen Teile, Bauteile oder Komponenten der Werkzeugelemente 14, 16, 16', 16", 16'" sowie 138 und 140 können insbesondere separat hergestellt und anschließend zusammengefügt werden, zum Beispiel durch Verkleben. Alternativ ist es zum Beispiel auch möglich, die elektrisch leitenden Teile der HF-Elektroden 28 und 29 als Einlegeteile in ein Kunststoffspritzwerkzeug einzulegen und mit einem Kunststoff zu umspritzen. Wie bereits erwähnt, können die Elektroden aus einem Metall oder einer elektrisch leitenden Keramik ausgebildet sein. Bei einer Segmentierung der HF-Elektroden 28 und 29 wie oben beschrieben, muss dann beispielsweise eine entsprechende Anzahl elektrisch leitender Elektrodensegmente aus einem Metall oder einer Metalllegierung oder einer elektrisch leitenden Keramik in das Kunststoffspritzwerkzeug vor Umspritzen mit einem geeigneten Kunststoff eingelegt werden.

Bei einer rein keramischen Ausführung der Werkzeugelemente 14, 16, 16', 16", 16'" sowie 138 und 140 bietet sich insbesondere ein Keramikpulverspritzgussverfahren an, beispielsweise die sogenannte "2K CIM"-Technologie, ein zwei Komponenten-Mikro-Keramikpulverspritzgussverfahren. Dabei werden zwei verschiedene Keramiken in einem Spritzprozess abgespritzt, die bei den fertigen Werkzeugelementen 14, 16, 16', 16", 16'" sowie 138 und 140 die elektrisch leitenden sowie elektrisch isolierenden Anteile ausbilden. Nach dem Abspritzen werden die zwei verschiedenen Keramiken zusammen gesintert. Dabei kann es sich zum Beispiel um eine Al₂O₃ Keramik und um eine Mischkeramik aus Al₂O₃ und TiN handeln.

## Patentansprüche

1. Chirurgisches Instrument (12) zum Verbinden von Körpergewebe mit einer Verbindungseinrichtung (20) zum Verbinden von Körpergewebe, wobei
die Verbindungseinrichtung (20) zwei relativ zueinander bewegbare Werkzeugelemente (14, 16) umfasst, wobei
das Instrument (12) eine Schneideinrichtung (86) mit einem Schneidelement (88) zum Durchtrennen von Gewebe umfasst und das Schneidelement (88) relativ zu mindestens einem der Werkzeugelemente (14, 16) bewegbar angeordnet ist,
**dadurch gekennzeichnet, dass**
die Schneideinrichtung (86) als eine HF-Schneideinrichtung (86) ausgebildet ist, die das Schneidelement (88) und eine diesem gegenüberliegende Gegenelektrode (96) aufweist, die mit HF-Strom beaufschlagbar sind;
die Gegenelektrode (96) an einem der Werkzeugelemente (14, 16) ausgebildet ist;
das Schneidelement (88) eine Schneidkante (90) aufweist, welche eine Schneidebene (92) definiert, die relativ zu einer Werkzeugelementfläche, an der die Gegenelektrode (96) ausgebildet ist, geneigt ist, wobei
die Schneidkante (90) des Schneidelements (88) und die Gegenelektrode (96) ringförmig ausgebildet sind.

2. Chirurgisches Instrument nach Anspruch 1, **dadurch gekennzeichnet, dass** das Schneidelement (88) relativ zur Gegenelektrode (96) um eine vom Instrument (12) im Bereich der Verbindungseinrichtung (20) definierten Längsachse (54) verdrehbar ist.

3. Chirurgisches Instrument nach Anspruch 1 oder 2, **dadurch**
**gekennzeichnet, dass** die Schneideinrichtung (86) in Form einer bipolaren Schneideinrichtung (86) ausgebildet ist.

4. Chirurgisches Instrument nach einem der voranstehenden Ansprüche,
**dadurch gekennzeichnet, dass** die Schneidkante (90) ringförmig geschlossen ist.

5. Chirurgisches Instrument nach einem der voranstehenden Ansprüche,
**dadurch gekennzeichnet, dass** das Instrument (12) mindestens einen mit der Schneideinrichtung (86) elektrisch leitend verbundenen Schneidanschluss (94) aufweist.

6. Chirurgisches Instrument nach Anspruch 5, **dadurch gekennzeichnet, dass** der mindestens eine Schneidanschluss (94) elektrisch leitend mit dem Schneidelement (86) verbunden ist.

7. Chirurgisches Instrument nach einem der voranstehenden Ansprüche,
**dadurch gekennzeichnet, dass** die Werkzeugelemente (14, 16) relativ zueinander verschwenkbar und/oder verschiebbar ausgebildet sind.

8. Chirurgisches Instrument nach einem der voranstehenden Ansprüche,
**dadurch gekennzeichnet, dass** das Instrument (12) eine Betätigungseinrichtung (76) umfasst zum Bewegen der Werkzeugelemente (14, 16) relativ zueinander.

9. Chirurgisches Instrument nach einem der voranstehenden Ansprüche,
**dadurch gekennzeichnet, dass** das Instrument (12) eine Schneidbetätigungseinrichtung (98) umfasst zum Bewegen des Schneidelements (88) und mindestens eines der Werkzeugelemente (14, 16) relativ zueinander.

10. Chirurgisches Instrument nach Anspruch 8 oder 9, **dadurch**
**gekennzeichnet, dass** die Betätigungseinrichtung (76) und/oder die Schneidbetätigungseinrichtung (98) an einem proximalen Ende des Instruments (12) angeordnet oder ausgebildet sind.

11. Chirurgisches Instrument nach einem der Ansprüche 8 bis 10, **dadurch gekennzeichnet, dass** die Betätigungseinrichtung (76) und/oder die Schneidbetätigungseinrichtung (98) zwei relativ zueinander verschwenkbare Betätigungsglieder (100) umfassen, welche mit mindestens einem der Werkzeugelemente (14, 16) oder dem Schneidelement (88) in Wirkverbindung stehen zum Übertragen einer Betätigungskraft zum Bewegen des mindestens einen Werkzeugelements (16) relativ zum anderen Werkzeugelement (14) oder des mindestens einen Werkzeugelements (14, 16) relativ zum Schneidelement (88).

12. Chirurgisches System mit einem chirurgischen Instrument nach einem der voranstehenden Ansprüche und mindestens einen HF-Stromgenerator (18), welcher mit den HF-Elektroden (28, 29) und/oder dem Schneidelement (88) wahlweise elektrisch leitend verbindbar ist.

## Claims

1. A surgical instrument (12) for connecting body tissue with a connecting means (20) for connecting body tissue, wherein
the connecting means (20) comprises two tool elements (14, 16) movable in relation to one another, wherein
the instrument (12) comprises a cutting means (86) with a cutting element (88) for cutting tissue and the cutting element (88) is movable in relation to at least one of the tool elements (14, 16),
**characterized in that**
the cutting means (86) is designed as an RF cutting means (86) that has the cutting element (88) and a counterelectrode (96) lying opposite same, to which RF current can be fed;
the counterelectrode (96) is formed on one of the tool elements (14, 16); and
the cutting element (88) has a cutting edge (90), which defines a cutting plane (902), which is sloped in relation to a tool element surface, on which the counterelectrode (96) is formed, wherein
the cutting edge (90) of the cutting element (88) and the counterelectrode (96) have a circular design.

2. A surgical instrument in accordance with claim 1, **characterized in that**
the cutting element (88) can be rotated in relation to the counterelectrode (96) about a longitudinal axis (54) defined by the instrument (12) in the area of the connecting means (20).

3. A surgical instrument in accordance with claim 1 or 2, **characterized in that** the cutting means (86) is designed in the form of a bipolar cutting means (86).

4. A surgical instrument in accordance with one of the preceding claims, **characterized in that** the cutting edge (90) has a self-contained circular design.

5. A surgical instrument in accordance with one of the preceding claims, **characterized in that** the instrument (12) has a least one cutting terminal (94) connected in an electrically conductive manner to the cutting means (86).

6. A surgical instrument in accordance with claim 5, **characterized in that** the at least one cutting terminal (94) is connected in an electrically conductive manner to the cutting element (86).

7. A surgical instrument in accordance with one of the preceding claims, **characterized in that** the tool elements (14, 16) are designed as pivotable and/or displaceable in relation to one another.

8. A surgical instrument in accordance with one of the preceding claims, **characterized in that** the instrument (12) comprises an actuation means (76) for moving the tool elements (14, 16) in relation to one another.

9. A surgical instrument in accordance with one of the preceding claims, **characterized in that** the instrument (12) comprises a cutting actuation means (98) for moving the cutting element (88) and at least one of the tool elements (14, 16) in relation to one another.

10. A surgical instrument in accordance with claim 8 or 9, **characterized in that** the actuation means (76) and/or the cutting actuation means (98) are arranged or formed at a proximal end of the instrument (12).

11. A surgical instrument in accordance with one of the claims 8 to 10, **characterized in that** the actuation means (76) and/or the cutting actuation means (98) comprise two actuation members (100) pivotable in relation to one another, which are in operative connection with at least one of the tool elements (14, 16) or the cutting element (88) for transmitting an actuation force for moving the at least one tool element (16) in relation to the other tool element (14) or the at least one tool element (14, 16) in relation to the cutting element (88).

12. A surgical system with a surgical instrument in accordance with one of the preceding claims and at least one RF current generator (18), which can be selectively connected in an electrically conductive manner to the RF electrodes (28, 29) and/or the cutting element (88).

## Revendications

1. Instrument chirurgical (12) pour réunir des tissus corporels, avec un dispositif de réunion (20) pour réunir des tissus corporels, dans lequel
le dispositif de réunion (20) comprend deux éléments d'outil (14, 16) mobiles l'un par rapport à l'autre, dans lequel
l'instrument (12) comprend un dispositif de coupe (86) avec un élément de coupe (88) pour couper des tissus et l'élément de coupe (88) est agencé mobile par rapport à au moins un des éléments d'outil (14, 16),
**caractérisé en ce que**
le dispositif de coupe (86) est réalisé comme un dispositif de coupe HF (86) qui comporte l'élément de coupe (88) et une électrode complémentaire (96) située à l'opposé de celui-ci qui peuvent être alimentés avec un courant HF ;
l'électrode complémentaire (96) est réalisée au niveau d'un des éléments d'outil (14, 16) ;
l'élément de coupe (88) comporte une arête de coupe (90) qui définit un plan de coupe (92) qui est incliné par rapport à une surface d'élément d'outil au niveau de laquelle l'électrode complémentaire (96) est réalisée, dans lequel
l'arête de coupe (90) de l'élément de coupe (88) et l'électrode complémentaire (96) sont réalisées en forme d'anneau.

2. Instrument chirurgical selon la revendication 1, **caractérisé en ce que** l'élément de coupe (88) peut être tourné par rapport à l'électrode complémentaire (96) autour d'un axe longitudinal (54) défini par l'instrument (12) dans la zone du dispositif de réunion (20).

3. Instrument chirurgical selon la revendication 1 ou 2, **caractérisé en ce que** le dispositif de coupe (86) est réalisé sous la forme d'un dispositif de coupe (86) bipolaire.

4. Instrument chirurgical selon l'une quelconque des revendications précédentes, **caractérisé en ce que** l'arête de coupe (90) est fermée en forme d'anneau.

5. Instrument chirurgical selon l'une quelconque des revendications précédentes, **caractérisé en ce que** l'instrument (12) comporte au moins un raccordement de coupe (94) relié de manière électriquement conductrice au dispositif de coupe (86).

6. Instrument chirurgical selon la revendication 5, **caractérisé en ce que** l'au moins un raccordement de coupe (94) est relié de manière électriquement conductrice à l'élément de coupe (86).

7. Instrument chirurgical selon l'une quelconque des revendications précédentes, **caractérisé en ce que** les éléments d'outil (14, 16) sont réalisés de manière à pouvoir être pivotés ou déplacés en translation l'un par rapport à l'autre.

8. Instrument chirurgical selon l'une quelconque des revendications précédentes, **caractérisé en ce que** l'instrument (12) comprend un dispositif d'actionnement (76) destiné à déplacer les éléments de coupe (14, 16) l'un par rapport à l'autre.

9. Instrument chirurgical selon l'une quelconque des revendications précédentes, **caractérisé en ce que** l'instrument (12) comprend un dispositif d'actionnement de coupe (98) destiné à déplacer l'élément de coupe (88) et au moins un des éléments d'outil (14, 16) l'un par rapport à l'autre.

10. Instrument chirurgical selon la revendication 8 ou 9, **caractérisé en ce que** le dispositif d'actionnement (76) et/ou le dispositif d'actionnement de coupe (98) sont agencés ou réalisés au niveau d'une extrémité proximale de l'instrument (12).

11. Instrument chirurgical selon l'une quelconque des revendications 8 à 10, **caractérisé en ce que** le dispositif d'actionnement (76) et/ou le dispositif d'actionnement de coupe (98) comprennent deux organes d'actionnement (100) qui peuvent pivoter l'un par rapport à l'autre et qui sont en liaison active avec au moins un des éléments d'outil (14, 16) ou l'élément de coupe (88) afin de transmettre une force d'actionnement destinée à déplacer l'au moins un élément d'outil (16) par rapport à l'autre élément d'outil (14) ou l'au moins un élément d'outil (14, 16) par rapport à l'élément de coupe (88).

12. Système chirurgical avec un instrument chirurgical selon l'une quelconque des revendications précédentes et avec au moins un générateur de courant HF (18) qui peut être relié sélectivement de manière électriquement conductrice aux électrodes HF (28, 29) et/ou à l'élément de coupe (88).
